# EUROPEAN PATENT APPLICATION

(11) **EP 3 547 247 A1**
(43) Date of publication of application: **02.10.2019**
(21) Application number: 18863817.5
(22) Date of filing: 30.07.2018
(51) Int. Cl.: G06Q 50/10, G06Q 50/22

(54) **SEARCH SYSTEM, INFORMATION PROVISION SYSTEM, CLIENT-SIDE DEVICE, HOST-SIDE DEVICE, INFORMATION PROVISION METHOD, INFORMATION PROVISION PROGRAM, CLIENT-SIDE PROGRAM, AND HOST-SIDE PROGRAM**

(30) Priority: 31.01.2018 JP 2018014698; 17.04.2018 JP 2018079165
(71) Applicant: Reasonwhy Inc., Tokyo 105-0001 (JP)
(72) Inventor: The designation of the inventor has not yet been filed
(74) Representative: Seppo Laine Oy
(86) International application number: PCT/JP2018/028436
(87) International publication number: WO 2019/150605

(57) **Abstract**

[Problems to be Solved by Inventions] To provide a search system, an information provision system, a client side device, a host side device, an information provision method, an information provision program, a client side program, and a host side program, which are capable of providing an optimal service to a client.

[Solutions to Solve Problems] A search system 1 includes a plurality of host side devices 10, a client side device 20, and an information provision device 30, the client side device 20 includes a client side communication unit, a client side output section, and an acquisition unit, the host side device 10 includes a host side communication unit, a host side output section, a proposal intention information generation unit generating proposal intention information, and a proposal information generation unit generating proposal information, and the information provision device 30 includes a communication unit, a host summary DB storing host identification information and host summary information, a provision unit providing various information items, and a notification information generation unit generating notification information.

## Description

### Technical Field

The present invention relates to a search system, an information provision system, a client side device, a host side device, an information provision method, an information provision program, a client side program, and a host side program.

### Background Art

In the related art, a technology has been proposed in which in a case where a fire station terminal receives an emergency notification from a patient terminal, an emergency hospital reservation system searches a hospital to accommodate a patient, on the basis of medical interview data of the patient, received from the fire station terminal, patient data managed by the emergency hospital reservation system, or information indicating an accommodation situation of the hospital, and a reservation is performed with respect to the searched hospital, as one of technologies for supporting a patient to receive a medical service (for example, refer to Patent Document 1).

### Citation List

### Patent Document

Patent Document 1: JP-A-2005-281

### Summary of the Invention

### Problems to be Solved by Inventions

Here, in the above-described technology of the related art, a medical doctor of the searched hospital to accommodate the patient, provides a medical service to the patient reserved by the emergency hospital reservation system, and for example, in a case where an excellent medical service of medical services that can be provided by the medical doctor, is different from the medical service provided to the patient, there is a possibility that it is difficult to provide an optimal medical service to the patient. Accordingly, there is a room for improvement, from the viewpoint of providing an optimal service to a client such as the patient.

The present invention has been made in consideration of the circumstances described above, and an object thereof is to provide a search system, an information provision system, a client side device, a host side device, an information provision method, an information provision program, a client side program, and a host side program, which are capable of providing an optimal service to a client.

### Solutions to Solve Problems

In order to solve the aforementioned problem and to achieve the object, a search system described in claim 1 is a search system searching a host providing a service to a client, the system comprises: a plurality of host side devices; a client side device; and an information provision system connected to each of the plurality of host side devices and the client side device to be capable of communicating with each other, wherein the client side device includes, a client side communication section for communicating with the information provision system or the plurality of host side devices, a client side output section outputting various information items relevant to the client side device, and an acquisition section acquiring diagnosis information indicating diagnosis contents of a diagnosis target, each of the plurality of host side devices includes, a host side communication section for communicating with the information provision system or the client side device, a host side output section outputting various information items relevant to each of the host side devices, a proposal intention information generation section generating proposal intention information indicating that there is an intention of performing a proposal of the service according to the diagnosis contents of the diagnosis target, and a proposal information generation section generating proposal information indicating proposal contents of the service, the information provision system includes, a communication section for communicating with the plurality of host side devices or the client side device, a host summary information storage section storing host identification information for uniquely identifying the host and host summary information indicating a summary of the host in association with each other, a provision section providing various information items to the plurality of host side devices or the client side device, and a notification information generation section generating notification information for notifying that it is a host performing the proposal of the service, the client side communication section of the client side device transmits the diagnosis information acquired by the acquisition section, to the information provision system, in a case in which the diagnosis information is received by the communication section, the provision section of the information provision system provides the diagnosis information to the plurality of host side devices, in a case in which a predetermined timing arrives after the diagnosis information provided by the provision section is output by the host side output section, the host side communication section of each of the plurality of host side devices transmits information including the proposal intention information generated by the proposal intention information generation section and the host identification information corresponding to the host side device to the information provision system, in a case in which the information including the proposal intention information and the host identification information is received by the communication section, the provision section of the information provision system extracts the host summary information corresponding to the host identification information from the host summary information stored in the host summary information storage section and provides information including the extracted host summary information and the corresponding host identification information to the client side device, the host summary information is output by the client side output section from the information including the host summary information and the host identification information provided by the provision section, and then, the client side communication section of the client side device transmits the host identification information corresponding to the host summary information specified by a predetermined method from the output host summary information to the information provision system, in a case in which the corresponding host identification information is received by the communication section, the provision section of the information provision system provides the notification information generated by the notification information generation section to the host side device corresponding to the host identification information, in a case in which a predetermined timing arrives after the notification information provided by the provision section is output by the host side output section, the host side communication section of each of the plurality of host side devices transmits the proposal information generated by the proposal information generation section to the information provision system, in a case in which the proposal information is received by the communication section, the provision section of the information provision system provides the proposal information to the client side device, and the proposal information is provided by the provision section, and then, the client side output section of the client side device outputs the proposal information.

Moreover, an information provision system described in claim 2 is an information provision system connected to each of a plurality of host side devices and a client side device to be capable of communicating with each other, the system comprises: a communication section for communicating with the plurality of host side devices or the client side device; a host summary information storage section storing host identification information for uniquely identifying a host providing a service to a client and host summary information indicating a summary of the host in association with each other; a provision section providing various information items to the plurality of host side devices or the client side device; and a notification information generation section generating notification information for notifying that it is a host performing a proposal of the service with respect to the client, wherein in a case in which diagnosis information indicating diagnosis contents of a diagnosis target is received by the communication section, the provision section provides the diagnosis information to the plurality of host side devices, in a case in which information including proposal intention information indicating that there is an intention of performing the proposal of the service according to the diagnosis contents of the diagnosis target and the host identification information is received by the communication section, the provision section extracts the host summary information corresponding to the host identification information from the host summary information stored in the host summary information storage section and provides information including the extracted host summary information and the corresponding host identification information to the client side device, in a case in which the host identification information corresponding to the host summary information specified by the client side device is received by the communication section, the provision section of the information provision system provides the notification information generated by the notification information generation section to the host side device corresponding to the host identification information, and in a case in which proposal information indicating proposal contents of the service is received by the communication section, the provision section of the information provision system provides the proposal information to the client side device.

Moreover, the information provision system described in claim 3 is the information provision system according to claim 2, wherein in a case in which information including the proposal intention information, compensation information indicating a compensation required at least for the proposal of the service or provision propriety information indicating whether or not to provide the proposed service to the client, and the host identification information is received by the communication section, the provision section extracts the host summary information corresponding to the received host identification information from the host summary information stored in the host summary information storage section and provides information including the extracted host summary information, the corresponding host identification information, and the received compensation information or the provision propriety information to the client side device.

Moreover, the information provision system described in claim 4 is the information provision system according to claim 2 or 3, wherein in a case in which questionnaire response information indicating a response of the host with respect to a service questionnaire is received by the communication section, the provision section provides the received questionnaire response information to the client side device.

Moreover, the information provision system described in claim 5 is the information provision system according to claim 4, further comprises: an aggregated information generation section generating aggregated information in which responses of a plurality of questionnaire response information items are aggregated in a case in which the plurality of questionnaire response information items are received by the communication section, wherein the plurality of questionnaire response information items are received, and then, the provision section provides the aggregated information generated by the aggregated information generation section to the client side device or the plurality of host side devices.

Moreover, the information provision system described in claim 6 is the information provision system according to claim 5, further comprises: a past questionnaire response information storage section storing the questionnaire response information received in the past, wherein in a case in which the questionnaire response information is received by the communication section, the aggregated information generation section generates past aggregated information in which the responses of the questionnaire response information items stored in the past questionnaire response information storage section are aggregated according to a predetermined item corresponding to the received questionnaire response information, and the questionnaire response information is received, and then, the provision section provides the past aggregated information generated by the aggregated information generation section to the client side device or the plurality of host side devices.

Moreover, the information provision system described in claim 7 is the information provision system according to any one of claims 2 to 6, further comprises: a host specification information storage section storing the host identification information and host specification information for specifying the host in association with each other, wherein the proposal information is provided, and then, in a case in which information including first request information of requesting provision of the host specification information and the host identification information corresponding to the proposal information is received by the communication section, the provision section provides host specification information corresponding to the host identification information from the host specification information stored in the host specification information storage section to the client side device.

Moreover, the information provision system described in claim 8 is the information provision system according to claim 7, wherein the host specification information is provided, and then, in a case in which information including second request information of requesting a reservation of provision of the service and the host identification information corresponding to the host specification information is received by the communication section, the provision section provides the second request information at least to the host side device corresponding to the host identification information, and the second request information is provided, and then, in a case in which response information indicating whether or not a reservation with respect to the second request information is received is received by the communication section, the provision section provides the response information at least to the client side device.

Moreover, the information provision system described in claim 9 is the information provision system according to any one of claims 2 to 8, wherein the service is a medical service, the diagnosis target is the client, and the diagnosis information includes information indicating a medical certificate of the client, information indicating a medical information provision document of the client, or information indicating a list of differential diagnosis of the client.

Moreover, the information provision system described in claim 10 is the information provision system according to any one of claims 2 to 9, wherein the host summary information includes actual performance information indicating actual performance of the host, evaluation information which becomes an index at the time of evaluating the host, or background information indicating a background of the host.

Moreover, a client side device described in claim 11 is a client side device connected to each of an information provision system and a plurality of host side devices to be capable of communicating with each other, the device comprises: a client side communication section for communicating with the information provision system or the plurality of host side devices; a client side output section outputting various information items relevant to the client side device; and an acquisition section acquiring diagnosis information indicating diagnosis contents of a diagnosis target, wherein the client side communication section transmits the diagnosis information acquired by the acquisition section, to the information provision system, host summary information is output by the client side output section from information which is provided from the information provision system and includes the host summary information indicating a summary of a host providing a service to a client and host identification information for uniquely identifying the host, and then, the client side communication section transmits the host identification information corresponding to the host summary information specified by a predetermined method, from the output host summary information, to the information provision system, and proposal information indicating proposal contents of the service is provided from the information provision system, and then, the client side output section outputs the proposal information.

Moreover, a host side device described in claim 12 is a host side device connected to a client side device and an information provision system to be capable of communicating with each other, the device comprises: a host side communication section for communicating with the information provision system or the client side device; a host side output section outputting various information items relevant to the host side device; a proposal intention information generation section generating proposal intention information indicating that there is an intention of performing a proposal of a service with respect to a client according to diagnosis contents of a diagnosis target; and a proposal information generation section generating proposal information indicating proposal contents of the service, wherein in a case in which a predetermined timing arrives after diagnosis information provided from the information provision system and indicating the diagnosis contents of the diagnosis target is output by the host side output section, the host side communication section transmits information including the proposal intention information generated by the proposal intention information generation section and host identification information corresponding to the host side device for uniquely identifying a host providing the service to a client to the information provision system, and in a case in which a predetermined timing arrives after notification information provided from the information provision system for notifying that it is a host performing proposal of the service with respect to the client is output by the host side output section, the host side communication section transmits the proposal information generated by the proposal information generation section to the information provision system.

Moreover, an information provision method described in claim 13 is an information provision method to be performed in an information provision system connected to each of a plurality of host side devices and a client side device to be capable of communicating with each other, the method comprises: a communication step of allowing a communication section to communicate with the plurality of host side devices or the client side device; a provision step of allowing a provision section to provide various information items to the plurality of host side devices or the client side device; and a notification information generation step of allowing a notification information generation section to generate notification information for notifying that it is a host performing a proposal of a service to a client, wherein in the provision step, in a case in which diagnosis information indicating diagnosis contents of a diagnosis target is received in the communication step, the diagnosis information is provided to the plurality of host side devices, in a case in which information including proposal intention information indicating that there is an intention of performing the proposal of the service according to the diagnosis contents of the diagnosis target and host identification information for uniquely identifying the host is received in the communication step, host summary information corresponding to the host identification information is extracted from the host summary information indicating a summary of the host, which is stored in a host summary information storage section, in association with the host identification information, and information including the extracted host summary information and the corresponding host identification information is provided to the client side device, in a case in which the host summary information specified by the client side device is received in the communication step, the notification information generated by the notification information generation section is provided to the host side device corresponding to the host summary information, and in a case in which proposal information indicating proposal contents of the service is received in the communication step, the proposal information is provided to the client side device.

Moreover, an information provision program method described in claim 14 is an information provision program to be executed in an information provision system connected to each of a plurality of host side devices and a client side device to be capable of communicating with each other, the program allowing a computer to function as: a communication section for communicating with the plurality of host side devices or the client side device; a host summary information storage section storing host identification information for uniquely identifying a host providing a service to a client and host summary information indicating a summary of the host in association with each other; a provision section providing various information items to the plurality of host side devices or the client side device; and a notification information generation section generating notification information for notifying that it is a host performing a proposal of a service with respect to a client, wherein in a case in which diagnosis information indicating diagnosis contents of a diagnosis target is received by the communication section, the provision section provides the diagnosis information to the plurality of host side devices, in a case in which information including proposal intention information indicating that there is an intention of performing the proposal of the service according to the diagnosis contents of the diagnosis target, and the host identification information is received by the communication section, the provision section extracts the host summary information corresponding to the host identification information from the host summary information stored in the host summary information storage section and provides information including the extracted host summary information and the corresponding host identification information to the client side device, in a case in which the host identification information corresponding to the host summary information specified by the client side device is received by the communication section, the provision section of the information provision system provides the notification information generated by the notification information generation section to the host side device corresponding to the host identification information, and in a case in which proposal information indicating proposal contents of the service is received by the communication section, the provision section of the information provision system provides the proposal information to the client side device.

Moreover, a client side program method described in claim 15 is a client side program to be executed in a client side device connected to each of an information provision system and a plurality of host side devices to be capable of communicating with each other, the program allowing a computer to function as: a client side communication section for communicating with the information provision system or the plurality of host side devices; a client side output section outputting various information items relevant to the client side device; and an acquisition section acquiring diagnosis information indicating diagnosis contents of a diagnosis target, wherein the client side communication section transmits the diagnosis information acquired by the acquisition section, to the information provision system, host summary information is output by the client side output section from information which is provided from the information provision system and includes the host summary information indicating a summary of a host providing a service to a client and host identification information for uniquely identifying the host, and then, the client side communication section transmits the host identification information corresponding to the host summary information specified by a predetermined method, from the output host summary information, to the information provision system, and proposal information indicating proposal contents of the service is provided from the information provision system, and then, the client side output section outputs the proposal information.

Moreover, a host side program method described in claim 16 is a host side program to be executed in a host side device connected to a client side device and an information provision system to be capable of communicating with each other, the program allowing a computer to function as: a host side communication section for communicating with the information provision system or the client side device; a host side output section outputting various information items relevant to the host side device; a proposal intention information generation section generating proposal intention information indicating that there is an intention of performing a proposal of a service with respect to a client according to diagnosis contents of a diagnosis target; and a proposal information generation section generating proposal information indicating proposal contents of the service, wherein in a case in which a predetermined timing arrives after diagnosis information provided from the information provision system and indicating the diagnosis contents of the diagnosis target is output by the host side output section, the host side communication section transmits information including the proposal intention information generated by the proposal intention information generation section, and host identification information which corresponds to the host side device for uniquely identifying a host providing the service to a client to the information provision system, and in a case in which a predetermined timing arrives after notification information which is provided from the information provision system for notifying that it is a host performing proposal of the service with respect to the client is output by the host side output section, the host side communication section transmits the proposal information generated by the proposal information generation section, to the information provision system.

### Advantageous Effect of the Invention

According to the search system described in claim 1, the information provision system in claim 2, the client side device in claim 11, the host side device described in claim 12, the information provision method described in claim 13, the information provision program described in claim 14, the client side program described in claim 15, and the host side program described in claim 16, in a case where the provision section of the information provision system provides the diagnosis information to the plurality of host side devices, and in a case where a predetermined timing arrives after the diagnosis information is output by the host side output section, the host side communication section of each of the plurality of host side devices transmits information including the proposal intention information, and the host identification information corresponding to the host side device, to the information provision device, and in a case where the information including the proposal intention information and the host identification information, is received, the provision section of the information provision device extracts the host summary information corresponding to the host identification information, from the host summary information storage section, and provides information including the extracted host summary information and the corresponding host identification information, to the client side device, and the host summary information is output by the client side output section, and then, the client side communication section of the client side device transmits the host identification information corresponding to the specified host summary information, to the information provision device from the output host summary information, by a predetermined method, and in a case where the corresponding host identification information is received, the provision section of the information provision device provides the notification information to the host side device corresponding to the host identification information, and in a case where a predetermined timing arrives after the notification information is output by the host side output section, the host side communication section of each of the plurality of host side devices transmits the proposal information to the information provision device, and in a case where the proposal information is received, the provision section of the information provision device provides the proposal information to the client side device, and the client side output section of the client side device outputs the proposal information after the proposal information is provided, and thus, it is possible to present various proposal information items to the client. Accordingly, it is possible for the client to receive the service corresponding to the proposal information which meets the needs, from various proposal information items, and it is possible to provide the optimal service to the client.

According to the information provision system described in claim 3, in a case where information including the compensation information or the provision propriety information, and the host identification information, is received, the provision section extracts the host summary information corresponding to the received host identification information, from the host summary information stored in the host summary information storage section, and provides information including the extracted host summary information, the corresponding host identification information, and the received compensation information or provision propriety information, to the client side device, and thus, it is possible to present the compensation information and the provision propriety information, included in the information such as a host summary, to the client, and to easily search the host who meets the needs of the client.

According to the information provision system described in claim 4, in a case where the questionnaire response information is received by the communication section, the provision section provides the received questionnaire response information to the client side device, and thus, it is possible to present the questionnaire response information reflecting the opinion with respect to the service of the host, to the client. Accordingly, it is possible for the client to easily search the host who meets the needs of the client, on the basis of the questionnaire response information, and thus, it is possible to prevent the provision of the service which does not meet the needs of a user, from being received, or the host from being searched again.

According to the information provision system described in claim 5, the plurality of questionnaire response information items are received, and then, the provision section provides the aggregated information generated by the aggregated information generation section, to the client side device or the plurality of host side devices, and thus, it is possible to present the aggregated information reflecting the opinion of the plurality of hosts, to the client. Accordingly, the aggregated information can be treated as the determination standard at the time of searching the host, and thus, it is possible for the client to easily search the host who meets the needs of the client, on the basis of the aggregated information. In addition, it is possible to present the aggregated information to the plurality of hosts, and it is possible for the plurality of hosts to objectively grasp a standing position with respect to the responses of the questionnaires of themselves.

According to the information provision system described in claim 6, the questionnaire response information is received, and then, the provision section provides the past aggregated information generated by the aggregated information generation section, to the client side device or the plurality of host side devices, and thus, it is possible to present the past aggregated information to the client, and to treat the past aggregated information as the determination standard at the time of searching the host, and therefore, it is possible for the client to easily search the host who meets the needs of the client, on the basis of the past aggregated information. In addition, it is possible to present the past aggregated information to the plurality of hosts, and it is possible for the plurality of hosts to more objectively grasp the standing position with respect to the responses of the questionnaires of themselves.

According to the information provision system described in claim 7, in a case where the proposal information is provided, and then, information including the first request information, and the host identification information corresponding to the proposal information, is received, the provision section provides the host specification information corresponding to the host identification information, to the client side device, from the host specification information stored in the host specification information storage section, and thus, it is possible to present the host specification information to the client, and for example, it is possible for the client to reliably contact with the host providing the service.

According to the information provision system described in claim 8, in a case where the host specification information is provided, and then, information including the second request information, and the host identification information corresponding to the host specification information, is received, the provision section provides the second request information at least to the host side device corresponding to the host identification information, and in a case where the second request information is provided, and then, the response information is received, the provision section provides the response information at least to the client side device, and thus, it is possible to present the response information at least to the client. Accordingly, it is possible to reserve the provision of the service without allowing the client to visit the facility providing the service, and to reliably receive the service from the host who provides the service.

According to the information provision system described in claim 9, the service is the medical service, the diagnosis target is the client, and the diagnosis information includes the information indicating the medical certificate of the client, the information indicating the medical information provision document of the client, or the information indicating the list of the differential diagnosis of the client, and thus, it is possible to present such information items to the host, and it is possible for the host to grasp the diagnosis contents of the client in detail.

According to the information provision system described in claim 10, the host summary information includes the actual performance information, the evaluation information, or the background information, and thus, it is possible to present such information items to the client, and to grasp the summary of the host in detail.

### Brief Description of the Drawings

Fig. 1 is a diagram conceptually illustrating a search system according to Embodiment 1 of the present invention.
Fig. 2 is a block diagram illustrating an electrical configuration of a host side device.
Fig. 3 is a block diagram illustrating an electrical configuration of a client side device.
Fig. 4 is a block diagram illustrating an electrical configuration of an information provision device.
Fig. 5 is a diagram exemplifying information stored in a host summary DB.
Fig. 6 is a diagram exemplifying information stored in a host specification DB.
Fig. 7 is a flowchart of search processing relevant to the client side device according to Embodiment 1.
Fig. 8 is a flowchart of search processing relevant to the information provision device according to Embodiment 1.
Fig. 9 is a flowchart of search processing relevant to the host side device according to Embodiment 1.
Fig. 10 is a block diagram illustrating an electrical configuration of an information provision device according to Embodiment 2.
Fig. 11 is a diagram exemplifying information stored in a past questionnaire response DB.

### Mode for Carrying Out the Invention

Hereinafter, embodiments of a search system, an information provision system, a client side device, a host side device, an information provision method, an information provision program, a client side program, and a host side program, according to the present invention, will be described in detail, with reference to the attached drawings.

### [Basic Concept of Embodiments]

First, the basic concept of embodiments will be described.

A search system according to the present invention is schematically a system searching a host providing a service to a client. Here, the "client" indicates a subject receiving the service provided from the host. In addition, the "host" indicates a subject providing the service to the client. In addition, the "service" indicates a service enjoyed by the client from the host, and for example, is a concept including a medical service, a consulting service, and the like. In addition, a device functioning as the search system is a device for searching the client, and for example, includes a plurality of devices connected in a wired or wireless manner, and specifically, includes a host side device, a client side device, and an information provision system. Among them, the "host side device" is a device possessed by the host, and for example, is a concept including a mobile terminal (as an example, a smart phone or a tablet terminal), a stationary personal computer, or the like. In addition, the "client side device" is a device possessed by the client, and for example, is a concept including a mobile terminal (as an example, a smart phone or a tablet terminal), a stationary personal computer, or the like. In addition, the "information provision system" is a system provided in a base station or the like, and for example, is a concept including a stationary server or the like. Hereinafter, in the embodiments, in a case where the service is a medical service, the case of searching a medical doctor (host) providing the medical service to a patient (client), will be described.

### [Specific Contents of Embodiments]

Subsequently, specific contents of the embodiments will be described.

### [Embodiment 1]

First, a search system according to Embodiment 1 will be described. Embodiment 1 is an aspect of providing proposal information described below to a client side device described below.

### (Configuration)

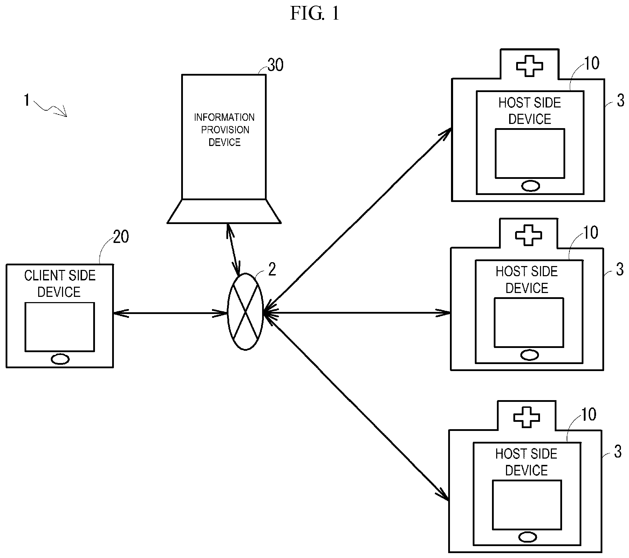

First, the configuration of the search system according to Embodiment 1 will be described. Fig. 1 is a diagram conceptually illustrating the search system according to Embodiment 1 of the present invention. As illustrated in Fig. 1, a search system 1 includes a plurality of host side devices 10, a client side device 20, and an information provision device 30, and the plurality of host side devices 10, the client side device 20, and the information provision device 30 are connected to be capable of communicating with each other, through a network 2. Furthermore, the configurations of the plurality of host side devices 10 can be identical to each other, and thus, hereinafter, the configuration of one host side device 10 will be described, and the description of the configurations of the other host side devices 10 will be omitted.

### (Configuration-Host Side Device)

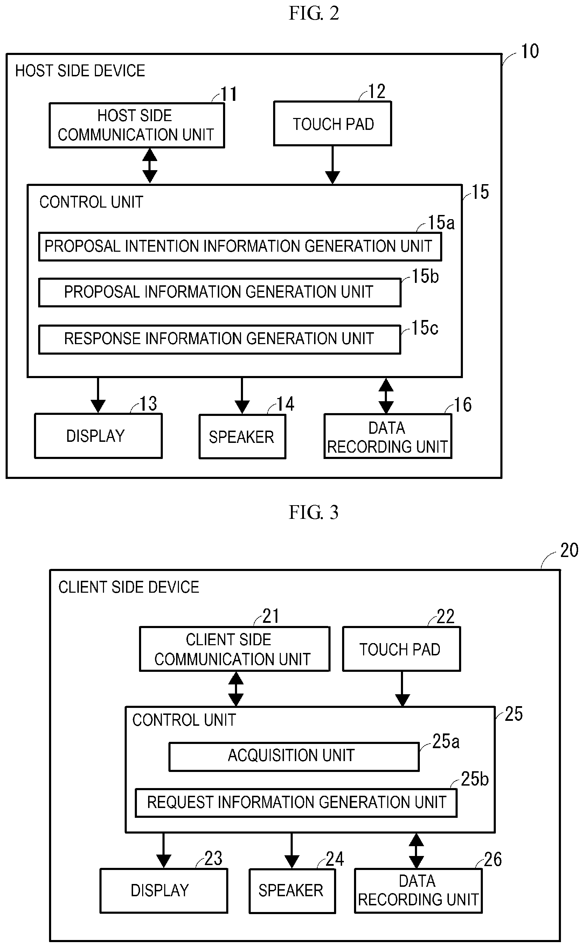

Next, the configuration of the host side device 10 will be described. Fig. 2 is a block diagram illustrating an electrical configuration of the host side device 10. The host side device 10 is a device transmitting proposal intention information described below, and as illustrated in Fig. 2, includes a host side communication unit 11, a touch pad 12, a display 13, a speaker 14, a control unit 15, and a data recording unit 16. Furthermore, the host side device 10, for example, is capable of including a known mobile terminal or the like, possessed by a medical doctor belonging to various medical facilities 3 illustrated in Fig. 1, and thus, the detailed description thereof will be omitted.

### (Configuration-Host Side Device-Host Side Communication Unit)

The host side communication unit 11 is a host side communication section for communicating with the information provision device 30 or the client side device 20, and for example, includes a known communication section performing communication by using a wired communication network or a mobile wireless communication network (furthermore, the same applies to a client side communication unit 21 of the client side device 20 described below, and a communication unit 31 of the information provision device 30 described below).

### (Configuration-Host Side Device-Touch Pad)

The touch pad 12 is a manipulation section receiving various manipulation inputs from the medical doctor, by being pressed with a finger or the like of the medical doctor. A specific configuration of the touch pad 12 is arbitrary, and for example, a known touch pad including a manipulation position detection section in resistive film type, electrostatic capacitance type, or the like can be used (furthermore, the same applies to a touch pad 22 of the client side device 20 described below).

### (Configuration-Host Side Device-Display)

The display 13 is a display section displaying various information items relevant to the host side device 10. A specific configuration of the display 13 is arbitrary, and for example, a flat panel display such as a known liquid crystal display or organic EL display, or the like can be used (furthermore, the same applies to a display 23 of the client side device 20 described below). Furthermore, the touch pad 12 and the display 13 may be integrally formed as the touch pad 12.

### (Configuration-Host Side Device-Speaker)

The speaker 14 is a sound output section outputting various information items relevant to the host side device 10, as a sound. A specific aspect of the sound output from the speaker 14 is arbitrary, and a synthetic sound generated as necessary, and a sound recorded in advance, can be output (furthermore, the same applies to a speaker 24 of the client side device 20 described below). Furthermore, the "display 13" and the "speaker 14" described above, correspond to the "host side output section" in the claims.

### (Configuration-Host Side Device-Control Unit)

The control unit 15 is a control section controlling the host side device 10, and is a computer including a CPU, various programs interpreted and executed on the CPU (including a basic control program such as an OS, and an application program of realizing a specific function activated on the OS), and an internal memory for storing a program or various data items, such as a RAM (furthermore, the same applies to a control unit 25 of the client side device 20 described below and a control unit 32 of the information provision device 30 described below). In particular, a host side program according to Embodiment 1 is installed in the host side device 10 through an arbitrary recording medium or the network 2, and thus, substantially configures each unit of the control unit 15.

In addition, as illustrated in Fig. 2, the control unit 15 functionally conceptually includes a proposal intention information generation unit 15a, a proposal information generation unit 15b, and a response information generation unit 15c.

The proposal intention information generation unit 15a is a proposal intention information generation section generating information indicating that there is an intention of performing a proposal of a service according to diagnosis contents of a diagnosis target (in Embodiment 1, the client himself/herself), with respect to the client (hereinafter, referred to as "proposal intention information").

The proposal information generation unit 15b is a proposal information generation section generating information indicating proposal contents of the service (in Embodiment 1, a medical treatment method, a medical treatment period, a medical treatment cost, and the like) (hereinafter, referred to as "proposal information").

The response information generation unit 15c is a response information generation section generating response information described below. Furthermore, the details of processing to be executed by the control unit 15 will be described below.

### (Configuration-Host Side Device-Data Recording Unit)

The data recording unit 16 is a recording section recording a program and various data items, necessary for operating the host side device 10 (for example, host identification information of the host side device 10, described below), and for example, includes a hard disk (not illustrated) as an external recording device. Here, a magnetic recording medium such as a magnetic disk, an optical recording medium such as a DVD or a Blu-ray disk, or an electrical recording medium such as a Flash Rom, a USB memory, and an SD card, are included instead of the hard disk or along with the hard disk, and the other arbitrary recording media can be used (furthermore, the same applies to a data recording unit 26 of the client side device 20 described below and a data recording unit 33 of the information provision device 30 described below).

### (Configuration-Client Side Device)

Next, the configuration of the client side device 20 will be described. Fig. 3 is a block diagram illustrating an electrical configuration of the client side device 20. The client side device 20 is a device transmitting diagnosis information described below, and as illustrated in Fig. 3, includes the client side communication unit 21, the touch pad 22, the display 23, the speaker 24, the control unit 25, and the data recording unit 26. Furthermore, the client side device 20, for example, is capable of including a known mobile terminal or the like possessed by a patient who regularly visits a medical facility (not illustrated) other than the medical facilities 3 illustrated in Fig. 1, and thus, the detailed description thereof will be omitted.

### (Configuration-Client Side Device-Client Side Communication Unit, Touch Pad, Display, and Speaker)

The client side communication unit 21 is a client side communication section for communicating with the information provision device 30 or the host side device 10. The touch pad 22 is a manipulation section receiving various manipulation inputs from the patient, by being pressed with a finger or the like of the patient. The display 23 is a display section displaying various information items relevant to the client side device 20. The speaker 24 is a sound output section outputting various information items relevant to the client side device 20, as a sound. Furthermore, the "display 23" and the "speaker 24" described above, correspond to the "client side output section" in the claims.

### (Configuration-Client Side Device-Control Unit)

The control unit 25 is a control section controlling the client side device 20, and specifically, a client side program according to Embodiment 1 is installed in the client side device 20 through an arbitrary recording medium or the network 2, and thus, substantially configures each unit of the control unit 25.

In addition, as illustrated in Fig. 3, the control unit 25 functionally conceptually includes an acquisition unit 25a and a request information generation unit 25b.

The acquisition unit 25a is an acquisition section acquiring information indicating the diagnosis contents of the diagnosis target (hereinafter, referred to as "diagnosis information"). Here, in Embodiment 1, the diagnosis information will be described as information including information indicating a medical certificate of the patient, information indicating a medical information provision document of the patient, information indicating a list of differential diagnosis of the patient, or the like.

The request information generation unit 25b is a request information generation section generating first request information and second request information, described below. Furthermore, the details of processing to be executed by the control unit 25 will be described below.

### (Configuration-Client Side Device-Data Recording Unit)

The data recording unit 26 is a recording section recording a program and various data items, necessary for operating the client side device 20 (for example, client identification information of the client side device 20, described below).

### (Configuration-Information Provision Device)

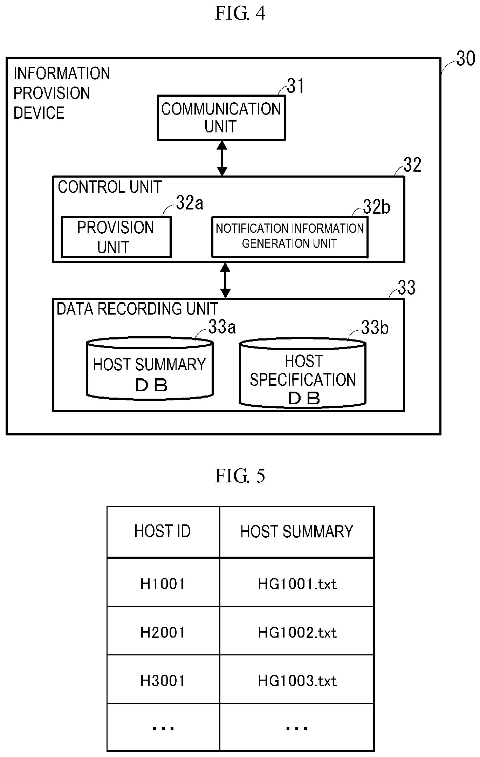

Next, the configuration of the information provision device 30 will be described. Fig. 4 is a block diagram illustrating an electrical configuration of the information provision device 30. As described below, the information provision device 30 is an information provision system performing the provision of the diagnosis information, or the like, and as illustrated in Fig. 4, includes the communication unit 31, the control unit 32, and the data recording unit 33. Furthermore, the information provision device 30, for example, is capable of including a known server or the like, provided in the base station (not illustrated), and thus, the detailed description thereof will be omitted.

### (Configuration-Information Provision Device-Communication Unit)

The communication unit 31 is a communication section for communicating with the plurality of host side devices 10 or the client side device 20.

### (Configuration-Information Provision Device-Control Unit)

The control unit 32 is a control section controlling the information provision device 30, and specifically, an information provision program according to Embodiment 1 is installed in the information provision device 30 through an arbitrary recording medium or the network 2, and thus, substantially configures each unit of the control unit 32.

In addition, as illustrated in Fig. 4, the control unit 32 functionally conceptually includes a provision unit 32a and a notification information generation unit 32b.

The provision unit 32a is a provision section providing various information items to the plurality of host side devices 10 or the client side device 20.

The notification information generation unit 32b is a notification information generation section generating information for notifying that it is a medical doctor performing the proposal of the service with respect to the patient (hereinafter, referred to as "notification information"). Furthermore, the details of processing to be executed by the control unit 32 will be described below.

### (Configuration-Information Provision Device-Data Recording Unit)

The data recording unit 33 is a recording section recording a program and various data items, necessary for operating the information provision device 30, and as illustrated in Fig. 4, includes a host summary database (hereinafter, the database will be referred to as an "DB") 33a, and a host specification DB 33b. Furthermore, an update method of information stored in the host summary DB 33a and the host specification DB 33b, is arbitrary, and for example, it is desirable that at least a part of the information stored in the host summary DB 33a and the host specification DB 33b, is updated, at a timing when information for updating the information stored in the host summary DB 33a or the host specification DB 33b from an external device, is acquired, a timing when a predetermined manipulation is performed by a manager of the information provision device 30, or the like, through a manipulation unit (not illustrated), or a timing when a certain period has elapsed.

### (Configuration-Information Provision Device-Data Recording Unit-Host Summary DB)

The host summary DB 33a is a host summary information storage section storing the host identification information and host summary information in association with each other. Here, the "host identification information" is information of specifying the medical doctor. In addition, the "host summary information" is information indicating the summary of the medical doctor, and in Embodiment 1, includes actual performance information, evaluation information, background information, and the like (here, the host summary information is not limited thereto, and for example, may include any one (or any two) of the actual performance information, the evaluation information, and the background information). Among them, the "actual performance information" is information indicating actual performance of the medical doctor, and for example, is a concept including the number of medical treatments information indicating the number of medical treatments performed with respect to the patient by the medical doctor, the number of complete recoveries information indicating the number of complete recoveries obtained by the medical treatment of the medical doctor, and the like. In addition, the "evaluation information" is information which becomes an index at the time of evaluating the medical doctor, and for example, in a case where the evaluation is set to five ranks, the evaluation is represented as "1", "2", "3", "4", "5", and the like. A setting method of the evaluation information is arbitrary, and for example, the evaluation information corresponding to the actual performance information stored in the host summary DB 33a, may be extracted from an evaluation table (not illustrated) which stores the actual performance information and the evaluation information in association with each other, and is stored in the data recording unit 33 (as an example, an evaluation table or the like, which stores Evaluation Information = "1" in a case where the number of medical treatments information of the actual performance information is "less than 1000", Evaluation Information = "2" in a case where the number of medical treatments information is "1000 cases to 3000 cases", Evaluation Information = "3" in a case where the number of medical treatments information is "3000 cases to 7000 cases", Evaluation Information = "4" in a case where the number of medical treatments information is "7000 cases to 10000 cases", Evaluation Information = "5" in a case where the number of medical treatments information is "greater than or equal to 10000 case", and the like), and the extracted information may be set as the evaluation information to be set. Alternatively, the average value of evaluation information items which are input by each of the patients, and are received from a plurality of client side devices 20, may be set as the evaluation information to be set. In addition, the "background information" is information indicating the background of the medical doctor, and for example, is a concept including work background information indicating a work background of the medical doctor, education background information indicating an education background of the medical doctor, and the like.

Fig. 5 is a diagram illustrating a configuration example of the host summary DB 33a. As illustrated in Fig. 5, the host summary DB 33a stores an item of "Host ID", an item of "Host Summary", and information corresponding to each of the items in association with each other. Among them, the information corresponding to the item of "Host ID" is the host identification information, and for example, "H1001" which is the ID of the medical doctor illustrated in Fig. 5, or the like corresponds to the information. The information corresponding to the item of "Host Summary" is the host summary information, and for example, "HG1001.txt" which is a file name of the host summary information (in Embodiment 1, a file name of a file which is stored in a text format, and includes the actual performance information, the evaluation information, and the background information) illustrated in Fig. 5, or the like corresponds to the information.

### (Configuration-Information Provision Device-Data Recording Unit-Host Specification DB)

The host specification DB 33b is a host specification information storage section storing the host identification information and the host specification information in association with each other. Here, the "host specification information" is information for specifying the medical doctor, and in Embodiment 1, the host specification information includes host name information, host address information, host email address information, and the like (here, the host specification information is not limited thereto, and for example, may include any one (or any two) of the host name information, the host address information, and the host email address information). Among them, the "host name information" is information indicating the name of the medical doctor. In addition, the "host address information" is information indicating the address of the medical doctor. In addition, the "host email address information" is information indicating an email address of the medical doctor.

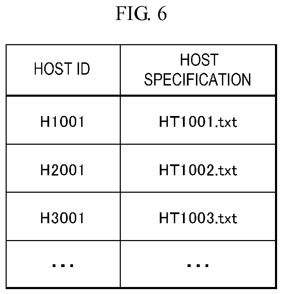

Fig. 6 is a diagram illustrating a configuration example of the host specification DB 33b. As illustrated in Fig. 6, the host specification DB 33b stores the item of "Host ID", an item of "Host Specification", and information corresponding to each of the items in association with each other. Among them, the information corresponding to the item of "Host ID" is the host identification information, and for example, "H1001" which is the ID of the medical doctor illustrated in Fig. 6, or the like corresponds to the information. The information corresponding to the item of "Host Specification" is the host specification information, and for example, "HT1001.txt" which is a file name of the host specification information illustrated in Fig. 6 (in Embodiment 1, a file name of a file which is stored in a text format, and includes the host name information, the host address information, and the host email address information), or the like corresponds to the information.

### (Processing)

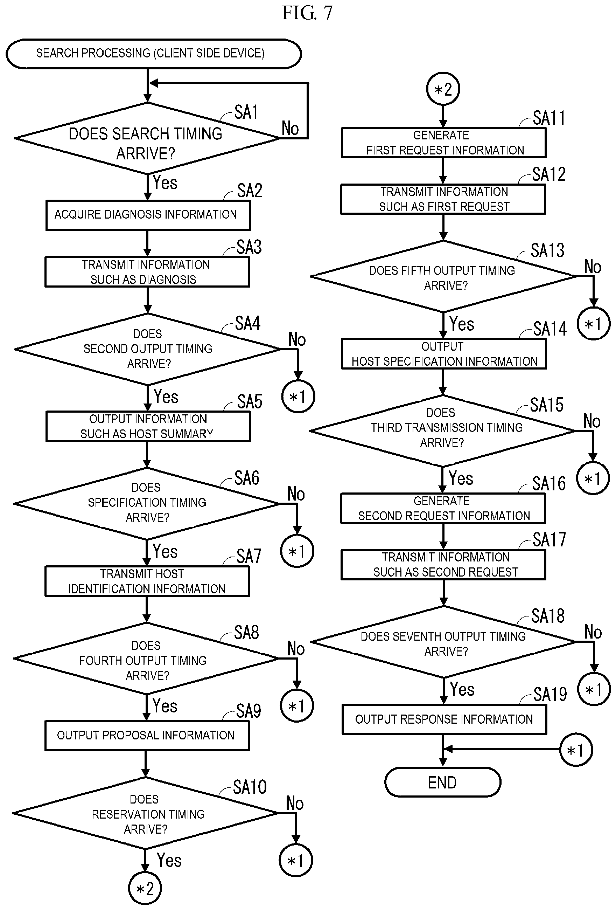

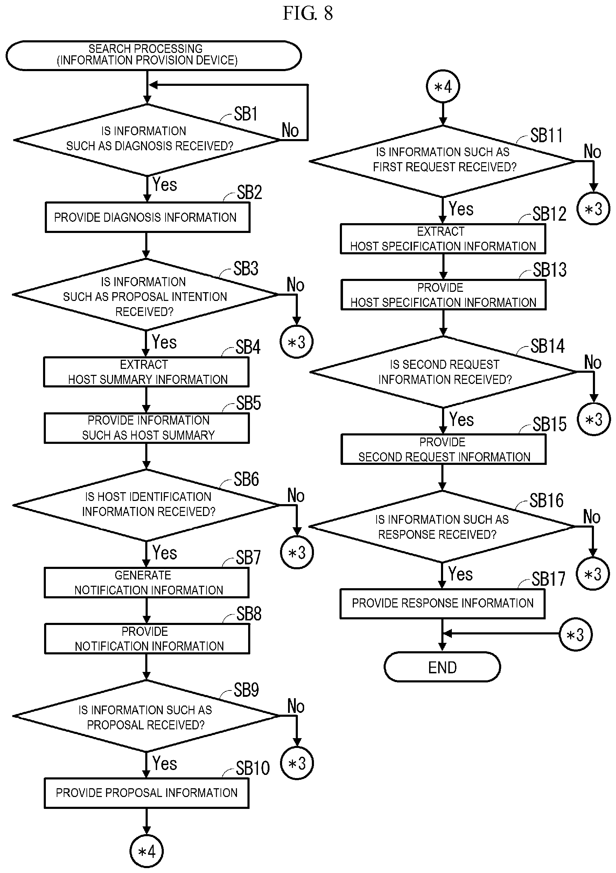

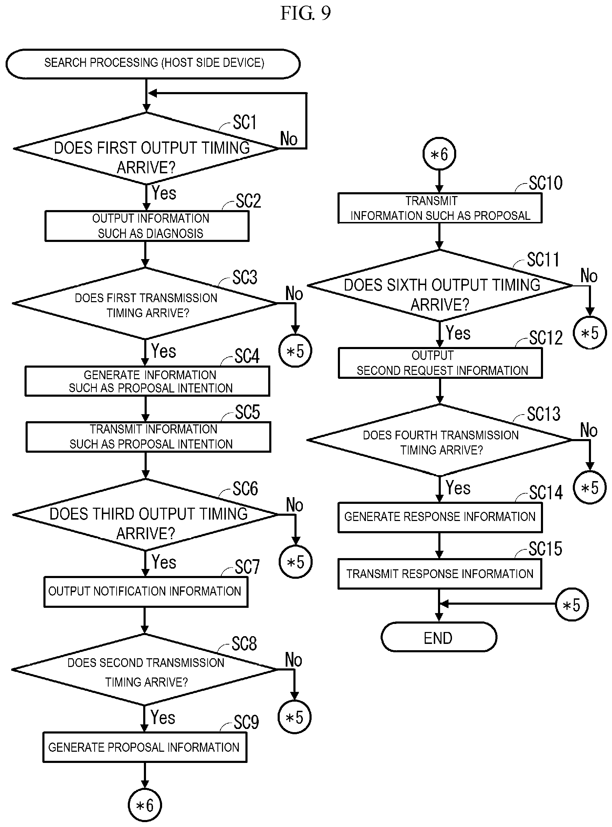

Next, search processing to be executed by the search system 1 configured as described above, will be described. Fig. 7 is a flowchart of search processing relevant to the client side device 20 according to Embodiment 1 (hereinafter, in the description of each processing, a step will be simply referred to as "S"). Fig. 8 is a flowchart of search processing relevant to the information provision device 30 according to Embodiment 1. Fig. 9 is a flowchart of search processing relevant to the host side device 10 according to Embodiment 1. The search processing is schematically processing of searching the medical doctor providing the service to the patient. In addition, a timing when the search processing is executed, is arbitrary, and in Embodiment 1, the timing will be described as a timing when the plurality of host side devices 10, the client side device 20, and the information provision device 30 are powered on, and then, are activated.

In a case where the search processing is activated, as illustrated in Fig. 7 to Fig. 9, the control unit 25 of the client side device 20 performs processings of SA1 to SA3, the control unit 32 of the information provision device 30 performs processings of SB1 and SB2, and the control unit 15 of the host side device 10 performs processings of SC1 and SC2, such that the diagnosis information can be presented to the medical doctor.

First, as illustrated in Fig. 7, in SA1, the control unit 25 of the client side device 20 determines whether or not a timing for searching the medical doctor (hereinafter, referred to as a "search timing") arrives. A determination method of whether or not the search timing arrives, is arbitrary, and for example, the determination is performed on the basis of whether or not a predetermined manipulation is performed by the patient through the touch pad 22, and in a case where the predetermined manipulation described above is performed, it is determined that the search timing arrives, and in a case where the predetermined manipulation described above is not performed, it is determined that the search timing does not arrive (furthermore, the same applies to the processing of SC1 described below). Then, the control unit 25 of the client side device 20 stands by until it is determined that the search timing arrives (SA1, No), and in a case where it is determined that the search timing arrives (SA1, Yes), the process proceeds to SA2.

In SA2, the acquisition unit 25a of the client side device 20 acquires the diagnosis information. An acquisition method of the diagnosis information is arbitrary, and for example, a predetermined manipulation may be performed by the patient through the touch pad 22, and thus, the diagnosis information recorded in advance in the data recording unit 26 may be acquired, or the diagnosis information may be acquired from the external device.

In SA3, the control unit 25 of the client side device 20 transmits information including the diagnosis information acquired in SA2 and the client identification information stored in the data recording unit 26 (hereinafter, referred to as "information such as diagnosis") to the information provision device 30, by the client side communication unit 21.

In addition, as illustrated in Fig. 8, in SB1, the control unit 32 of the information provision device 30 determines whether or not the information such as diagnosis transmitted in SA3, is received by the communication unit 31. Then, the control unit 32 of the information provision device 30 stands by until the information such as diagnosis is received (SB1, No), and in a case where the information such as diagnosis is received (SB1, Yes), the process proceeds to SB2.

In SB2, the provision unit 32a of the information provision device 30 provides the diagnosis information included in the information such as diagnosis, received in SB1, to the plurality of host side devices 10 (for example, the plurality of host side devices 10 corresponding to the host identification information stored in the host summary DB 33a). A provision method of the diagnosis information is arbitrary, and in Embodiment 1, the diagnosis information is provided by being uploaded to a website which can be accessed by the client side device 20, the plurality of host side devices 10, and the other external device, and is provided on the internet, in which information posted on the website can be browsed. Here, the provision method is not limited thereto, and for example, the diagnosis information may be provided by being transmitted to the plurality of host side devices 10 by an email (furthermore, the same applies to processings of SB5, SB8, SB10, SB13, SB15, and SB17, described below).

In addition, as illustrated in Fig. 9, in SC1, the control unit 15 of the host side device 10 determines whether or not a timing for outputting the diagnosis information (hereinafter, referred to as a "first output timing") arrives. Then, the control unit 15 of the host side device 10 stands by until it is determined that the first output timing arrives (SC1, No), and in a case where it is determined that the first output timing arrives (SC1, Yes), the process proceeds to SC2.

In SC2, the control unit 15 of the host side device 10 outputs the diagnosis information provided in SB2, by the display 13 or the speaker 14. An output method of the diagnosis information is arbitrary, and for example, a display region (hereinafter, referred to as a "diagnosis display region") is generated on a screen of the display 13, and the diagnosis information is continuously or intermittently displayed on the generated diagnosis display region, or the diagnosis information is continuously or intermittently output by the speaker 14, as a sound.

According to the processing as described above, it is possible to present the diagnosis information to the medical doctor, and it is possible for the medical doctor to grasp the diagnosis contents of the patient. In addition, the diagnosis information includes the information indicating the medical certificate of the patient, the information indicating the medical information provision document of the patient, and the information indicating the list of differential diagnosis of the patient, and thus, it is possible to present such information items to the medical doctor, and it is possible for the medical doctor to grasp the diagnosis contents of the patient in detail. In particular, the information indicating the list of differential diagnosis of the patient is included, and thus, it is possible to prevent a diagnosis leakage or a diagnostic error of the medical doctor.

Next, as illustrated in Fig. 7 to Fig. 9, the control unit 25 of the client side device 20 performs processings of SA4 and SA5, the control unit 32 of the information provision device 30 performs processings of SB3 to SB5, and the control unit 15 of the host side device 10 performs processings of SC3 to SC5, such that the host summary information can be presented to the patient.

First, as illustrated in Fig. 9, in SC3, the control unit 15 of the host side device 10 determines whether or not a timing for transmitting the proposal intention information (hereinafter, referred to as a "first transmission timing") arrives. A determination method of whether or not the first transmission timing arrives, is arbitrary, and a display region (hereinafter, referred to as a "first transmission display region") is generated on the screen of the display 13, information indicating that there is an intention of performing the proposal of the service according to the diagnosis contents, and information indicating that there is no intention of performing the proposal of the service according to the diagnosis contents, are displayed on the generated first transmission display region, and the determination is performed on the basis of whether or not any one of such information items is selected by the medical doctor within a predetermined period (for example, within one week, within one month, or the like), through the touch pad 12. Here, in a case where the information indicating that there is the intention of performing the proposal of the service described above, is selected within a predetermined period, it is determined that the first transmission timing arrives, and in a case where the information indicating that there is no intention of performing the proposal of the service described above, is selected, or in a case where a selection manipulation is not received even when the predetermined period has elapsed, it is determined that the first transmission timing does not arrive. Then, in a case where the control unit 15 of the host side device 10 determines that the first transmission timing arrives (SC3, Yes), the process proceeds to SC4, and in a case where the control unit 15 of the host side device 10 determines that the first transmission timing does not arrive (SC3, No), the search processing is ended.

In SC4, the proposal intention information generation unit 15a of the host side device 10 generates the proposal intention information, compensation information, and provision propriety information. Here, the "compensation information" indicates information indicating a compensation required at least for the proposal of the service, and in Embodiment 1, is described as information indicating a charge in which a usage fee of the website described above is added to the compensation required for the proposal of the service, but is not limited thereto, and for example, may be information indicating only the compensation required for the proposal of the service. In addition, the "provision propriety information" indicates information indicating whether or not the service is provided to the patient.

In addition, a generation method of the proposal intention information and the compensation information is arbitrary, and for example, standard message information stored in advance in the data recording unit 16 (for example, text information or the like) may be generated as the proposal intention information or the compensation information, or information input by the medical doctor through the touch pad 12 may be generated as the proposal intention information or the compensation information. As an example, text information such as "Hope to perform the proposal of the medical service with respect to the client" is generated as the proposal intention information. In addition, a generation method of the provision propriety information is arbitrary, and for example, a display region (hereinafter, referred to as a "provision propriety generation display region") may be generated on the screen of the display 13, information indicating that the service is capable of being provided, and information indicating that the service is not capable of being provided, may be displayed on the generated provision propriety generation display region, and information in which any one of such information items is selected by the medical doctor through the touch pad 12, may be generated as the provision propriety information.

In SC5, the control unit 15 of the host side device 10 transmits information including the proposal intention information, the compensation information, and the provision propriety information, generated in SC4, and the host identification information stored in the data recording unit 16 (hereinafter, referred to as "information such as a proposal intention"), to the information provision device 30, by the host side communication unit 11.

Returning to Fig. 8, in SB3, the control unit 32 of the information provision device 30 determines whether or not the information such as a proposal intention, transmitted in SC5, is received within a predetermined period (for example, within one week, within one month, or the like), by the communication unit 31. Then, in a case where the control unit 32 of the information provision device 30 receives information such as a proposal intention within the predetermined period (SB3, Yes), the process proceeds to SB4, and in a case where the control unit 32 of the information provision device 30 does not receive the information such as a proposal intention within the predetermined period (SB3, No), the search processing is ended.

In SB4, the provision unit 32a of the information provision device 30 extracts the host summary information corresponding to the host identification information included in the information such as a proposal intention, which is received in SB3, from the host summary information stored in the host summary DB 33a.

In SB5, the provision unit 32a of the information provision device 30 provides information including the host summary information extracted in SB4, and the compensation information, the provision propriety information, and the host identification information included in the information such as a proposal intention transmitted in SC5 (hereinafter, referred to as "information such as a host summary"), to the client side device 20.

Returning to Fig. 7, in SA4, the control unit 25 of the client side device 20 determines whether or not a timing for outputting the host summary information (hereinafter, referred to as a "second output timing") arrives. A determination method of whether or not the second output timing arrives, is arbitrary, and the determination is performed on the basis of whether or not a predetermined manipulation is performed by the patient within a predetermined period (for example, within one week, within one month, or the like), through the touch pad 22, and in a case where the predetermined manipulation described above is performed, it is determined that the second output timing arrives, and in a case where the predetermined manipulation described above is not performed, it is determined that the second output timing does not arrive (furthermore, the same applies to processings of SA4, SA8, SA13, SA18, SC6, SC8, SC11, and SC13, described below). Then, in a case where the control unit 25 of the client side device 20 determines that the second output timing arrives (SA4, Yes), the process proceeds to SA5, and in a case where the control unit 25 of the client side device 20 determines that the second output timing does not arrive (SA4, No), the search processing is ended.

In SA5, the control unit 25 of the client side device 20 outputs the information such as a host summary, provided in SB5, by the display 23 or the speaker 24. An output method of the information such as a host summary is arbitrary, and for example, a display region (hereinafter, referred to as a "host summary display region") is generated on a screen of the display 23, the information such as a host summary is continuously or intermittently displayed on the generated host summary display region, or the information such as a host summary is continuously or intermittently output by the speaker 24, as a sound.

According to the processing as described above, it is possible to present the host summary information to the patient, and it is possible for the patient to grasp the summary of the medical doctor having the intention of performing the proposal of the service. In particular, the host summary information includes the actual performance information, the evaluation information, and the background information, and thus, it is possible to present such information items to the patient, and it is possible to grasp the summary of the medical doctor in detail. In addition, it is possible to present the compensation information and the provision propriety information, included in the information such as a host summary, to the patient, and it is possible to easily search the medical doctor who meets the needs of the patient.

Next, as illustrated in Fig. 7 to Fig. 9, the control unit 25 of the client side device 20 performs processings of SA6 and SA7, the control unit 32 of the information provision device 30 performs processings of SB6 to SB8, and the control unit 15 of the host side device 10 performs processings of SC6 and SC7, such that the notification information can be presented to the medical doctor.

First, as illustrated in Fig. 7, in SA6, the control unit 25 of the client side device 20 determines whether or not a timing for specifying desired host summary information from the host summary information included in the information such as a host summary, output in SA5 (hereinafter, referred to as a "specification timing") arrives. A determination method of whether or not the specification timing arrives, is arbitrary, and for example, the determination is performed on the basis of whether or not at least any one of the host summary information items displayed on the host summary display region described above is specified by the patient within a predetermined period (for example, within one week, within one month, or the like), through the touch pad 22. Here, in a case where the specification described above is performed, it is determined that the specification timing arrives, and in a case where the specification described above is not performed, it is determined that the specification timing does not arrive. Then, in a case where the control unit 25 of the client side device 20 determines that the specification timing arrives (SA6, Yes), the process proceeds to SA7, and in a case where the control unit 25 of the client side device 20 determines that the specification timing does not arrive (SA6, No), the search processing is ended.

In SA7, the control unit 25 of the client side device 20 transmits the host identification information corresponding to the host summary information specified in SA6 from the host identification information included in the information such as a host summary, provided in SB5, to the information provision device 30, by the client side communication unit 21.

In addition, as illustrated in Fig. 8, in SB6, the control unit 32 of the information provision device 30 determines whether or not the host identification information transmitted in SA7, is received within a predetermined period (for example, within one week, within one month, or the like), by the communication unit 31. Then, in a case where the control unit 32 of the information provision device 30 receives the host identification information within the predetermined period (SB6, Yes), the process proceeds to SB7, and in a case where the control unit 32 of the information provision device 30 does not receive the host identification information within the predetermined period (SB6, No), the search processing is ended. Furthermore, a proceeding method to SB7 is arbitrary, and for example, in a case where information indicating that the compensation of the compensation information corresponding to the host summary information is paid by the patient, is received in addition to the reception of the host identification information, the process may proceed to SB7.

In SB7, the notification information generation unit 32b of the information provision device 30 generates the notification information. A generation method of the notification information is arbitrary, and for example, the standard message information stored in advance in the data recording unit 33 may be generated as the notification information. As an example, text information such as "There is an offer from the client to request the proposal of the medical service with respect to xxx", is generated as the notification information (furthermore, in "xxx", the host identification information received in SB6, the host name information corresponding to the host identification information, or the like is incorporated).

In SB8, the provision unit 32a of the information provision device 30 provides the notification information generated in SB7, to the host side device 10 corresponding to the host identification information received in SB6.

In addition, as illustrated in Fig. 9, in SC6, the control unit 15 of the host side device 10 determines whether or not a timing for outputting the notification information (hereinafter, referred to as a "third output timing") arrives. Then, in a case where the control unit 15 of the host side device 10 determines that the third output timing arrives (SC6, Yes), the process proceeds to SC7, and in a case where the control unit 15 of the host side device 10 determines that the third output timing does not arrive (SC6, No), the search processing is ended.

In SC7, the control unit 15 of the host side device 10 outputs the notification information provided in SB8, by the display 13 or the speaker 14. An output method of the notification information is arbitrary, and for example, a display region (hereinafter, referred to as a "notification display region") is generated on the screen of the display 13, and the notification information is continuously or intermittently displayed on the generated notification display region, or the notification information is continuously or intermittently output by the speaker 14, as a sound.

According to the processing as described above, it is possible to present the notification information to the medical doctor, and it is possible for the medical doctor to grasp that the medical doctor himself/herself is selected as the medical doctor performing the proposal of the service with respect to the patient.

Next, as illustrated in Fig. 7 to Fig. 9, the control unit 25 of the client side device 20 performs processings of SA8 and SA9, the control unit 32 of the information provision device 30 performs processings of SB9 and SB10, and the control unit 15 of the host side device 10 performs processings of SC8 to SC10, such that the proposal information can be presented to the patient.

First, as illustrated in Fig. 9, in SC8, the control unit 15 of the host side device 10 determines whether or not a timing for transmitting the proposal information (hereinafter, referred to as a "second transmission timing") arrives. Then, in a case where the control unit 15 of the host side device 10 determines that the second transmission timing arrives (SC8, Yes), the process proceeds to SC9, and in a case where the control unit 15 of the host side device 10 determines that the second transmission timing does not arrive (SC8, No), the search processing is ended.

In SC9, the proposal information generation unit 15b of the host side device 10 generates the proposal information. A generation method of the proposal information is arbitrary, and for example, information input by the medical doctor, through the touch pad 12, may be generated as the proposal information, or information acquired from the external device, may be generated as the proposal information.

In SC10, the control unit 15 of the host side device 10 transmits information including the proposal information generated in SC9, and the host identification information stored in the data recording unit 16 (hereinafter, referred to as "information such as a proposal"), to the information provision device 30, by the host side communication unit 11.

Returning to Fig. 8, in SB9, the control unit 32 of the information provision device 30 determines whether or not the information such as a proposal, transmitted in SC10, is received within a predetermined period (for example, within one week, within one month, or the like), by the communication unit 31. Then, in a case where the control unit 32 of the information provision device 30 receives the information such as a proposal within the predetermined period (SB9, Yes), the process proceeds to SB10, and in a case where the control unit 32 of the information provision device 30 does not receive the information such as a proposal within the predetermined period (SB9, No), the search processing is ended. Furthermore, for example, the process proceeds to SB10, and then, the control unit 32 of the information provision device 30 may execute processing relevant to the payment of the compensation with respect to the medical doctor corresponding to the host identification information included in the information such as a proposal.

In SB10, the provision unit 32a of the information provision device 30 provides the proposal information included in the information such as a proposal, received in SB9, to the client side device 20.

Returning to Fig. 7, in SA8, the control unit 25 of the client side device 20 determines whether or not a timing for outputting the proposal information (hereinafter, referred to as a "fourth output timing") arrives. Then, in a case where the control unit 25 of the client side device 20 determines that the fourth output timing arrives (SA8, Yes), the process proceeds to SA9, and in a case where the control unit 25 of the client side device 20 determines that the fourth output timing does not arrive (SA8, No), the search processing is ended.

In SA9, the control unit 25 of the client side device 20 outputs the proposal information provided in SB10 by the display 23 or the speaker 24. An output method of the proposal information is arbitrary, and for example, a display region (hereinafter, referred to as a "proposal display region") is generated on the screen of the display 23, and the proposal information is continuously or intermittently displayed on the generated proposal display region, or the proposal information is continuously or intermittently output by the speaker 24, as a sound.

According to the processing as described above, it is possible to present various proposal information items to the patient. Accordingly, it is possible for the patient to receive the service corresponding to the proposal information which meets the needs, from various proposal information items, and it is possible to provide the optimal service to the patient.

Next, as illustrated in Fig. 7 and Fig. 8, the control unit 25 of the client side device 20 performs processings of SA10 to SA14, and the control unit 32 of the information provision device 30 performs processings of SB11 to SB13, such that the host specification information can be presented to the patient.

First, as illustrated in Fig. 7, in SA10, the control unit 25 of the client side device 20 determines whether or not a timing for reserving the provision of the service of the medical doctor corresponding to the proposal information output in SA9 (hereinafter, referred to as a "reservation timing") arrives. Then, in a case where the control unit 25 of the client side device 20 determines that the reservation timing arrives (SA10, Yes), the process proceeds to SA11, in a case where the control unit 25 of the client side device 20 determines that the reservation timing does not arrive (SA10, No), the search processing is ended.

In SA11, the request information generation unit 25b of the client side device 20 generates information of requesting the provision of the host specification information (hereinafter, referred to as "first request information"). A generation method of the first request information is arbitrary, and for example, the standard message information stored in advance in the data recording unit 26, is generated as the first request information.

In SA12, the control unit 25 of the client side device 20 transmits information including the first request information generated in SA11 and the host identification information corresponding to proposal information output in SA9 (specifically, the host identification information corresponding to all or a part (for example, a part specified by a predetermined method) of the proposal information output in SA9) (hereinafter, referred to as "information such as a first request"), to the information provision device 30, by the client side communication unit 21.

In addition, as illustrated in Fig. 8, in SB11, the control unit 32 of the information provision device 30 determines whether or not the information such as a first request, transmitted in SA12, is received within a predetermined period (for example, within one week, within one month, or the like), by the communication unit 31. Then, in a case where the control unit 32 of the information provision device 30 receives the information such as a first request within the predetermined period (SB11, Yes), the process proceeds to SB12, and in a case where the control unit 32 of the information provision device 30 dose not receive the information such as a first request within the predetermined period (SB11, No), the search processing is ended.

In SB12, the provision unit 32a of the information provision device 30 extracts the host specification information corresponding to the host identification information included in the information such as a first request, which is received in SB11, from the host specification information stored in the host specification DB 33b.

In SB13, the provision unit 32a of the information provision device 30 provides the host specification information extracted in SB12, to the client side device 20. Furthermore, a provision method of the host specification information is arbitrary, and for example, in a case where information indicating that the additional charge is paid by the patient, is received in addition to the reception of the information such as a first request, the host specification information may be provided.

Returning to Fig. 7, in SA13, the control unit 25 of the client side device 20 determines whether or not a timing for outputting the host specification information (hereinafter, referred to as a "fifth output timing") arrives. Then, in a case where the control unit 25 of the client side device 20 determines that the fifth output timing arrives (SA13, Yes), the process proceeds to SA14, and in a case where the control unit 25 of the client side device 20 determines that the fifth output timing does not arrive (SA13, No), the search processing is ended.

In SA14, the control unit 25 of the client side device 20 outputs the host specification information provided in SB13, by the display 23 or the speaker 24. An output method of the host specification information is arbitrary, and for example, a display region (hereinafter, referred to as a "host specification display region") is generated on the screen of the display 23, and the host specification information is continuously or intermittently displayed on the generated host specification display region, or the host specification information is continuously or intermittently output by the speaker 24, as a sound. In this case, for example, from the host specification information provided in SB13, host specification information of the medical doctor corresponding to information indicating that the service of the provision propriety information provided in SB5 is not capable of being provided, may not be output.

According to the processing as described above, it is possible to present the host specification information to the patient, and it is possible for the patient to reliably contact with the medical doctor providing the service. Furthermore, for example, after the processing of SA14, in a case where information indicating low evaluation with respect to an action when the patient contacts with a desired medical doctor, is received by the information provision device 30, the control unit 32 of the information provision device 30 may set the host side device 10 corresponding to the received information such that the host side device 10 is not capable of executing the search processing. As an example, the access to the website described above from the host side device 10 corresponding to the received information described above, may be rejected.

Next, as illustrated in Fig. 7 to Fig. 9, the control unit 25 of the client side device 20 performs processings of SA15 to SA17, the control unit 32 of the information provision device 30 performs processings of SB14 and SB15, and the control unit 15 of the host side device 10 performs processings of SC11 and SC12, such that second request information described below can be presented to the medical doctor.

First, as illustrated in Fig. 7, in SA15, the control unit 25 of the client side device 20 determines whether or not a timing for transmitting the second request information (hereinafter, referred to as a "third transmission timing") arrives. Then, in a case where the control unit 25 of the client side device 20 determines that the third transmission timing arrives (SA15, Yes), the process proceeds to SA16, and in a case where the control unit 25 of the client side device 20 determines that the third transmission timing does not arrive (SA15, No), the search processing is ended.

In SA16, the request information generation unit 25b of the client side device 20 generates information of requesting the reservation of the provision of the service (hereinafter, referred to as "second request information"). The second request information, for example, is a concept including information indicating a provision date of the service, information indicating a provision location of the service, and the like. In addition, a generation method of the second request information is arbitrary, and for example, information input by the patient through the touch pad 22, may be generated as the second request information.

In SA17, the control unit 25 of the client side device 20 transmits information including the second request information generated in SA16, and the host identification information corresponding to the host specification information output in SA14 (specifically, the host identification information corresponding to all or a part of the host specification information output in SA14) (hereinafter, referred to as "information such as a second request"), to the information provision device 30, by the client side communication unit 21.

In addition, as illustrated in Fig. 8, in SB14, the control unit 32 of the information provision device 30 determines whether or not the information such as a second request, transmitted in SA17, is received within a predetermined period (for example, within one week, within one month, or the like), by the communication unit 31. Then, in a case where the control unit 32 of the information provision device 30 receives the information such as a second request within the predetermined period (SB14, Yes), the process proceeds to SB15, and in a case where the control unit 32 of the information provision device 30 dose not receive the information such as a second request within the predetermined period (SB14, No), the search processing is ended.

In SB15, the provision unit 32a of the information provision device 30 provides the second request information included in the information such as a second request, received in SB14, to the host side device 10 corresponding to the host identification information included in the information such as a second request (or a device relevant to the host side device 10 (for example, a device or the like possessed by a person who assists the medical doctor (as an example, a nurse or the like)), in addition to the host side device 10). Furthermore, a provision method of the information such as a second request is arbitrary, and for example, in a case where the information indicating that the additional charge is paid by the patient, is received in addition to the reception of the information such as a second request, the information such as a second request may be provided.

In addition, as illustrated in Fig. 9, in SC11, the control unit 15 of the host side device 10 determines whether or not a timing for outputting the second request information (hereinafter, referred to as a "sixth output timing") arrives (furthermore, in SB15, in a case where the second request information is provided to the device relevant to the host side device 10 described above, approximately the same processing is performed in the device). Then, in a case where the control unit 15 of the host side device 10 determines that the sixth output timing arrives (SC11, Yes), the process proceeds to SC12, in a case where the control unit 15 of the host side device 10 determines that the sixth output timing does not arrive (SC11, No), the search processing is ended.

In SC12, the control unit 15 of the host side device 10 outputs the second request information provided in SB15, by the display 13 or the speaker 14 (furthermore, in SB15, in a case where the second request information is provided to the device relevant to the host side device 10, approximately the same processing is performed in the device). An output method of the second request information is arbitrary, and for example, a display region (hereinafter, referred to as a "request display region") is generated on the screen of the display 13, and the second request information is continuously or intermittently displayed on the generated request display region, or the second request information is continuously or intermittently output by the speaker 14, as a sound.

According to the processing as described above, it is possible to present the second request information at least to the medical doctor, and to inquire the medical doctor providing the service, about the reservation.

Subsequently, as illustrated in Fig. 7 to Fig. 9, the control unit 25 of the client side device 20 performs processings of SA18 and SA19, the control unit 32 of the information provision device 30 performs processings of SB16 and SB17, and the control unit 15 of the host side device 10 performs processings of SC13 to SC15, such that response information described below can be presented to the patient.

First, as illustrated in Fig. 9, in SC13, the control unit 15 of the host side device 10 determines whether or not a timing for transmitting the response information described below (hereinafter, referred to as a "fourth transmission timing") arrives. Then, in a case where the control unit 15 of the host side device 10 determines that the fourth transmission timing arrives (SC13, Yes), the process proceeds to SC14, and in a case where the control unit 15 of the host side device 10 determines that the fourth transmission timing does not arrive (SC13, No), the search processing is ended.

In SC14, the response information generation unit 15c of the host side device 10 generates information indicating whether or not the reservation with respect to the second request information output in SC12, is received (hereinafter, referred to as "response information"). A generation method of the response information is arbitrary, and for example, a display region (hereinafter, referred to as a "response generation display region") may be generated on the screen of the display 13, information indicating that the reservation is received, and information indicating that the reservation is not received, may be displayed on the generated response generation display region, any one of such information items may be generated on the basis of information selected by the medical doctor through the touch pad 12. As an example, text information such as "The reservation of the provision of the service is received", is generated as the response information.

In SC15, the control unit 15 of the host side device 10 transmits information including the response information generated in SC14, and the host identification information stored in the data recording unit 16 (hereinafter, referred to as "information such as a response"), to the information provision device 30, by the host side communication unit 11, and then, the search processing is ended.

Returning to Fig. 8, in SB16, the control unit 32 of the information provision device 30 determines whether or not the information such as a response, transmitted in SC15, is received within a predetermined period (for example, within one week, within one month, or the like), by the communication unit 31. Then, in a case where the control unit 32 of the information provision device 30 receives the information such as a response within the predetermined period (SB16, Yes), the process proceeds to SB17, in a case where the control unit 32 of the information provision device 30 does not receive the information such as a response within the predetermined period (SB 16, No), the search processing is ended.

In SB17, the provision unit 32a of the information provision device 30 provides the response information included in the information such as a response, received in SB16, to the client side device 20 (or the device relevant to the host side device 10, in addition to the client side device 20).

Returning to Fig. 7, in SA18, the control unit 25 of the client side device 20 determines whether or not a timing for outputting the response information (hereinafter, referred to as a "seventh output timing") arrives (furthermore, in SB17, in a case where the response information is provided to the device relevant to the host side device 10, approximately the same processing is performed in the device). Then, in a case where the control unit 25 of the client side device 20 determines that the seventh output timing arrives (SA18, Yes), the process proceeds to SA19, and in a case where the control unit 25 of the client side device 20 determines that the seventh output timing does not arrive (SA18, No), the search processing is ended.

In SA19, the control unit 25 of the client side device 20 outputs the response information provided in SB17, by the display 23 or the speaker 24 (furthermore, in SB17, in a case where the response information is provided to the device relevant to the host side device 10, approximately the same processing is performed in the device), and then, the search processing is ended. An output method of the response information is arbitrary, and for example, a display region (hereinafter, referred to as a "response display region") is generated on the screen of the display 23, and the response information is continuously or intermittently displayed on the generated response display region, or the proposal information is continuously or intermittently output by the speaker 24, as a sound.

According to the processing as described above, it is possible to present the response information at least to the patient. Accordingly, it is possible to reserve the provision of the service without allowing the patient to visit the facility providing the service (for example, a hospital or the like), and to reliably receive the service from the medical doctor who provides the service.

### (Effect of Embodiment 1)

According to Embodiment 1, in a case where the provision unit 32a of the information provision device 30 provides the diagnosis information to the plurality of host side devices 10, and in a case where a predetermined timing arrives after the diagnosis information is output by the host side output section, the host side communication unit 11 of each of the plurality of host side devices 10 transmits information including the proposal intention information, and the host identification information corresponding to the host side device 10, to the information provision device 30, and in a case where the information including the proposal intention information and the host identification information, is received, the provision unit 32a of the information provision device 30 extracts the host summary information corresponding to the host identification information, from the host summary DB 33a, and provides information including the extracted host summary information and the corresponding host identification information, to the client side device 20, and the host summary information is output by the client side output section, and then, the client side communication unit 21 of the client side device 20 transmits the host identification information corresponding to the specified host summary information, to the information provision device 30 from the output host summary information, by a predetermined method, and in a case where the corresponding host identification information is received, the provision unit 32a of the information provision device 30 provides the notification information to the host side device 10 corresponding to the host identification information, and in a case where a predetermined timing arrives after the notification information is output by the host side output section, the host side communication unit 11 of each of the plurality of host side devices 10 transmits the proposal information to the information provision device 30, and in a case where the proposal information is received, the provision unit 32a of the information provision device 30 provides the proposal information to the client side device 20, and the client side output section of the client side device 20 outputs the proposal information after the proposal information is provided, and thus, it is possible to present various proposal information items to the client. Accordingly, it is possible for the client to receive the service corresponding to the proposal information which meets the needs, from various proposal information items, and it is possible to provide the optimal service to the client.

In addition, in a case where information including the compensation information or the provision propriety information, and the host identification information, is received, the provision unit 32a extracts the host summary information corresponding to the received host identification information, from the host summary information stored in the host summary DB 33a, and provides information including the extracted host summary information, the corresponding host identification information, and the received compensation information or provision propriety information, to the client side device 20, and thus, it is possible to present the compensation information and the provision propriety information, included in the information such as a host summary, to the client, and to easily search the host who meets the needs of the client.

In addition, in a case where the proposal information is provided, and then, information including the first request information, and the host identification information corresponding to the proposal information, is received, the provision unit 32a provides the host specification information corresponding to the host identification information, to the client side device 20, from the host specification information stored in the host specification DB 33b, and thus, it is possible to present the host specification information to the client, and for example, it is possible for the client to reliably contact with the host providing the service.

In addition, in a case where the host specification information is provided, and then, information including the second request information, and the host identification information corresponding to the host specification information, is received, the provision unit 32a provides the second request information at least to the host side device 10 corresponding to the host identification information, and in a case where the second request information is provided, and then, the response information is received, the provision unit 32a provides the response information at least to the client side device 20, and thus, it is possible to present the response information at least to the client. Accordingly, it is possible to reserve the provision of the service without allowing the client to visit the facility providing the service, and to reliably receive the service from the host who provides the service.

In addition, the service is the medical service, the diagnosis target is the client, and the diagnosis information includes the information indicating the medical certificate of the client, the information indicating the medical information provision document of the client, or the information indicating the list of the differential diagnosis of the client, and thus, it is possible to present such information items to the host, and it is possible for the host to grasp the diagnosis contents of the client in detail.

In addition, the host summary information includes the actual performance information, the evaluation information, or the background information, and thus, it is possible to present such information items to the client, and to grasp the summary of the host in detail.

### [Embodiment 2]

First, a search system according to Embodiment 2 will be described. Embodiment 2 is an aspect of providing questionnaire response information described below to the client side device. Here, unless otherwise noted, the configuration of Embodiment 2 is approximately identical to the configuration of Embodiment 1, and the same reference numerals and/or names as those used in Embodiment 1, will be applied to the same configurations as those of Embodiment 1, as necessary, and the description thereof will be omitted.

### (Configuration)

First, the configuration of the search system according to Embodiment 2 will be described. The search system 1 according to Embodiment 2 has approximately the same configuration as that of the search system 1 according to Embodiment 1. Here, the details of the configuration of the information provision device 30 are devised as follows.

### (Configuration-Information Provision Device)

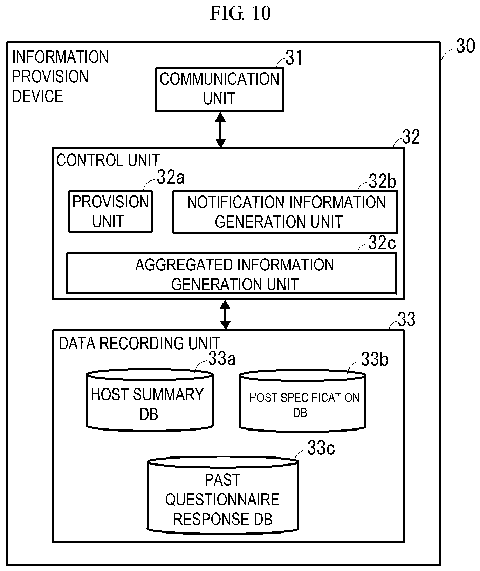

Fig. 10 is a block diagram illustrating an electrical configuration of the information provision device 30. As illustrated in Fig. 10, the information provision device 30 includes the communication unit 31, the control unit 32, and the data recording unit 33.

### (Configuration-Information Provision Device-Control Unit)

As illustrated in Fig. 10, the control unit 32 functionally conceptually includes the provision unit 32a, the notification information generation unit 32b, and an aggregated information generation unit 32c.

Among them, the aggregated information generation unit 32c is an aggregated information generation section generating aggregated information in a case where a plurality of questionnaire response information items are received by the communication unit 31. Here, the "questionnaire response information" is information indicating a response that the host performs with respect to a questionnaire relevant to the service. In Embodiment 2, the questionnaire response information corresponds to information indicating that the policy of the currently represented medical service is basically agreed, information indicating that the policy of the currently represented medical service is approximately agreed, but other choices can be considered, information indicating that the policies other than the policy of the currently represented medical service can be considered, information indicating the others, and the like. In addition, the "aggregated information" is information in which the responses of a plurality of questionnaire response information items are aggregated.

### (Configuration-Information Provision Device-Data Recording Unit)

As illustrated in Fig. 10, the data recording unit 33 includes the host summary DB 33a, the host specification DB 33b, and a past questionnaire response DB 33c.

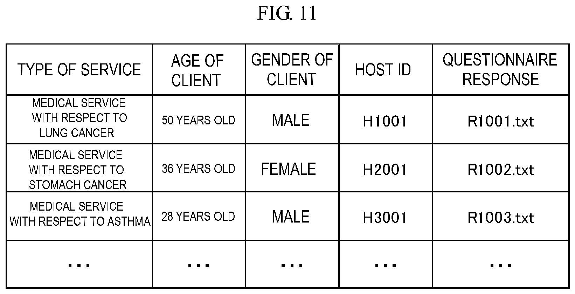

Among them, the past questionnaire response DB 33c is a past questionnaire response information storage section storing the questionnaire response information received in the past. Fig. 11 is a diagram illustrating a configuration example of the past questionnaire response DB 33c. As illustrated in Fig. 11, the past questionnaire response DB 33c stores an item of "Type of Service", an item of "Age of Client", an item of "Gender of Client", an item of "Host ID", an item of "Questionnaire Response", and information corresponding to each of the items in association with each other. Among them, the information corresponding to the item of "Type of Service" is service type information indicating the type of service, and for example, corresponds to "Medical Service with respect to Lung Cancer" or the like, which is the type of service illustrated in Fig. 11 (specifically, a medical service corresponding to the type of illness). In addition, the information corresponding to the item of "Age of Client", is client age information indicating the age of the client, and for example, corresponds to "50 years old" or the like, which is the age of the client illustrated in Fig. 11. In addition, the information corresponding to the item of "Gender of Client", is client gender information indicating the gender of the client, and for example, corresponds to "Male" or the like, which is the gender of the client illustrated in Fig. 11. In addition, the information corresponding to the item of "Host ID" is the host identification information. In addition, the information corresponding to the item of "Questionnaire Response", is the questionnaire response information, and for example, corresponds to "R1001.txt" which is a file name of the questionnaire response information illustrated in Fig. 11 (in Embodiment 2, a file name of a file which is stored in a text format, and includes the information indicating that the policy of the currently represented medical service is basically agreed, and the like), or the like.

Furthermore, in Embodiment 2, it is described that the past questionnaire response DB 33c stores the service type information, the client age information, the client gender information, and the host identification information, but the past questionnaire response DB 33c is not limited thereto. For example, the service type information may be information indicating the type of medical procedure of the medical service (as an example, information indicating an operative therapy of the cancer, information indicating a chemotherapy of the cancer, information indicating an actinotherapy of the cancer, and the like). In addition, the service type information may be information indicating the type of client, other than the client age information and the client gender information, and as an example, may be client identification information that the client uniquely identifies, or client location information indicating a resident area of the client. In addition, the service type information may be information indicating the type of host, other than the host identification information, and as an example, may be host medical facility information indicating the medical facility to which the host belongs, and host location information indicating a location where the medical facility to which the host belongs, exists.

### (Search Processing)

Next, search processing to be executed by the search system 1 configured as described above, will be described. The search processing according to Embodiment 2, is identical to SA1 to SA4 and SA6 to SA19 of Fig. 7, SB1 to SB4 and SB6 to SB17 of Fig. 8, SC1 to SC3 and SC6 to SC15 of Fig. 9, and thus, the illustration and the description thereof will be omitted.

First, as illustrated in Fig. 9, in SC4, the proposal intention information generation unit 15a of the host side device 10 generates the proposal intention information, the compensation information, the provision propriety information, and the questionnaire response information. Here, a generation method of the questionnaire response information is arbitrary, and for example, a display region (hereinafter, referred to as a "questionnaire response display region") is generated on the screen of the display 13, the information indicating that the policy of the currently represented medical service is basically agreed, the information indicating that the policy of the currently represented medical service is approximately agreed, but other choices can be considered, the information indicating that the policies other than the policy of the currently represented medical service can be considered, and the information indicating the others, are displayed on the generated questionnaire response display region, and when any one of such information items is selected by the medical doctor through the touch pad 12, within a predetermined time, the selected information is generated as the questionnaire response information to be generated.

In SC5, the control unit 15 of the host side device 10 transmits the information such as a proposal intention, including the proposal intention information, the compensation information, the provision propriety information, and the questionnaire response information, generated in SC4, and the host identification information stored in the data recording unit 16, to the information provision device 30, by the host side communication unit 11.

In addition, as illustrated in Fig. 8, in SB5, the provision unit 32a of the information provision device 30 provides the information such as a host summary, including the host summary information extracted in SB4, the compensation information, the provision propriety information, the host identification information, and the questionnaire response information, which are included in the information such as a proposal intention, transmitted in SC5, to the client side device 20. Accordingly, it is possible to present the questionnaire response information reflecting the opinion about the service of the host, to the client side device 20. Accordingly, the client is capable of easily searching the host who meets the needs of the client, on the basis of the questionnaire response information, and thus, it is possible to prevent the provision of the service which does not meet the needs of a user, from being received, or the host from being searched again.

Here, it is not limited thereto, and for example, the information such as a host summary, including the aggregated information, may be provided to the client side device 20, in addition to the information described above. A generation method of the aggregated information is arbitrary, and for example, in a case where a plurality of questionnaire response information items are received by the communication unit 31, the responses of the plurality of questionnaire response information items are aggregated by the aggregated information generation unit 32c (specifically, aggregated for each type of response), and information indicating the number of responses aggregated for each type of response, or a rate thereof, is generated as the aggregated information to be generated. As an example, in a case where ten questionnaire response information items are received, five (or 50%) information items indicating that the policy of the currently represented medical service is basically agreed, two (or 20%) information items indicating that the policy of the currently represented medical service is approximately agreed, but other choices can be considered, three (or 30%) information items indicating that the policies other than the policy of the currently represented medical service can be considered, and zero (or 0%) information items indicating the others, are generated. Accordingly, it is possible to present the aggregated information reflecting the opinion of a plurality of hosts, to the client side device 20. Accordingly, the aggregated information can be treated as a determination standard at the time of searching the host, and thus, it is possible for the client to easily search the host who meets the needs of the client, on the basis of the aggregated information. Furthermore, such aggregated information may be provided not only to the client side device 20, and for example, but also to the plurality of host side devices 10. Accordingly, it is possible to present the aggregated information to the plurality of hosts (for example, the plurality of hosts transmitting the questionnaire response information, or the like), and it is possible for the plurality of hosts to objectively grasp a standing position with respect to the responses of the questionnaires of themselves.

Alternatively, the information such as a host summary, further including past aggregated information, may be provided to the client side device 20. Here, in a case where the questionnaire response information is received by the communication unit 31, the "past aggregated information" indicates information in which the responses of the questionnaire response information items stored in the past questionnaire response DB 33c, are aggregated according to a predetermined item corresponding to the received questionnaire response information (for example, the type of service, the age of the client, the gender of the client, the host, or the like). In addition, a generation method of the past aggregated information is arbitrary, and for example, in a case where the questionnaire response information is received by the communication unit 31, the responses of the questionnaire response information items stored in the past questionnaire response DB 33c, are aggregated (specifically, aggregated for each type of response), according to a predetermined item corresponding to the received questionnaire response information (as an example, one item, or a combination of a plurality of items), and information indicating the number of responses aggregated for each type of response, or a rate thereof, is generated as the past aggregated information to be generated. As an example, in a case where the predetermined item corresponding to the questionnaire response information is the medical service with respect to the lung cancer, and in a case where there are 50 questionnaire response information items corresponding to the predetermined item, in the past questionnaire response DB 33c, 20 (or 40%) information items indicating that the policy of the currently represented medical service is basically agreed, 15 (or 30%) information items indicating that the policy of the currently represented medical service is approximately agreed, but other choices can be considered, ten (or 20%) information items indicating that the policies other than the policy of the currently represented medical service can be considered, and five (or 10%) information items indicating the others, are generated. Accordingly, it is possible to present the past aggregated information to the client side device 20, and to treat the past aggregated information as the determination standard at the time of searching the host, and thus, it is possible for the client to easily search the host who meets the needs of the client, on the basis of the past aggregated information. Furthermore, such past aggregated information may be provided not only to the client side device 20, and for example, but also to the plurality of host side devices 10. Accordingly, it is possible to present the past aggregated information to the plurality of hosts (for example, the plurality of hosts transmitting the questionnaire response information, or the like), and it is possible for the plurality of hosts to more objectively grasp the standing position with respect to the responses of the questionnaires of themselves.

In addition, as illustrated in Fig. 7, in SA5, the control unit 25 of the client side device 20 outputs the information such as a host summary, provided in SB5, by the display 23 or the speaker 24. Here, a display method of the information such as a host summary, is arbitrary, and for example, the host summary information, the compensation information, the provision propriety information, the host identification information, and the questionnaire response information, included in the information such as a host summary, are displayed on the host summary display region of the display 23 in association with each other. In addition, in a case where the aggregated information or the past aggregated information is included in the information such as a host summary, a display method of the aggregated information or the past aggregated information, is arbitrary, and for example, the aggregated information or the past aggregated information may be displayed on the host summary display region of the display 23, in a graph format (as an example, a circle graph format, a bar graph format, or the like) or in a table format.

### (Effect of Embodiment 2)

According to Embodiment 2, in a case where the questionnaire response information is received by the communication unit 31, the provision unit 32a provides the received questionnaire response information to the client side device 20, and thus, it is possible to present the questionnaire response information reflecting the opinion with respect to the service of the host, to the client. Accordingly, it is possible for the client to easily search the host who meets the needs of the client, on the basis of the questionnaire response information, and thus, it is possible to prevent the provision of the service which does not meet the needs of a user, from being received, or the host from being searched again.

In addition, the plurality of questionnaire response information items are received, and then, the provision unit 32a provides the aggregated information generated by the aggregated information generation unit 32c, to the client side device 20 or the plurality of host side devices 10, and thus, it is possible to present the aggregated information reflecting the opinion of the plurality of hosts, to the client. Accordingly, the aggregated information can be treated as the determination standard at the time of searching the host, and thus, it is possible for the client to easily search the host who meets the needs of the client, on the basis of the aggregated information. In addition, it is possible to present the aggregated information to the plurality of hosts, and it is possible for the plurality of hosts to objectively grasp a standing position with respect to the responses of the questionnaires of themselves.

In addition, the questionnaire response information is received, and then, the provision unit 32a provides the past aggregated information generated by the aggregated information generation unit 32c, to the client side device 20 or the plurality of host side devices 10, and thus, it is possible to present the past aggregated information to the client, and to treat the past aggregated information as the determination standard at the time of searching the host, and therefore, it is possible for the client to easily search the host who meets the needs of the client, on the basis of the past aggregated information. In addition, it is possible to present the past aggregated information to the plurality of hosts, and it is possible for the plurality of hosts to more objectively grasp the standing position with respect to the responses of the questionnaires of themselves.

### (Modification Examples of Embodiments)

Hereinbefore, the embodiments of the present invention have been described, but specific configurations and sections of the present invention can be arbitrarily modified and improved within the scope of the technical idea of each invention described in the claims. Hereinafter, such modification examples will be described.

### (Regarding to Technical Problems and Advantageous Effects of the Invention)

First, the technical problems and the advantageous effects of the invention are not limited to the above description, and there is a possibility that the technical problems and the advantageous effects of the invention are different according to an implementation environment of the invention or the details of the configuration, and there is a case where only a part of the technical problems described above is attained, or only a part of the advantageous effects described above is obtained.

### (Regarding to Distribution or Integration)

Each electrical constituent described above has been functionally conceptually described, and it is not necessary to have the configuration as physically illustrated. That is, a specific aspect of the distribution or the integration of each of the units is not limited to that of illustrated, and all or a part of thereof can be functionally or physically distributed or integrated in arbitrary unit, according to various loads, usage situations or the like. In the present application, the "system" is not limited to a system including a plurality of devices, and includes a system including a single device. In addition, in the present application, the "device" is not limited to a device including a single device, and includes a device including a plurality of devices. For example, the information provision device 30 may be configured by being distributed to a plurality of devices communicating with each other, the control unit 32 may be provided in a part of the plurality of devices, and the data recording unit 33 may be provided in the other part of the plurality of devices.

### (Regarding to Shape, Numerical Value, Structure, and Temporal Sequence)

In the constituents exemplified in the embodiments or the drawings, a mutual relationship in the shape, the numerical value, and the structure or the temporal sequence of the plurality of constituents, can be arbitrarily modified and improved, within the scope of the technical idea of the present invention.

### (Regarding to Diagnosis Target)

In Embodiments 1 and 2 described above, it has been described that the diagnosis target is the client (specifically, the patient), but the diagnosis target is not limited thereto, and for example, may be a person other than the client, a possession of the client (as an example, a pet, electronic goods, a vehicle, or the like), or the like.

### (Regarding to Host Side Device)

In Embodiments 1 and 2 described above, it has been described that the host side device 10 is possessed by the medical doctor, but the host side device 10 is not limited thereto. For example, the host side device 10 may be possessed by a nurse of the medical facility 3 to which the medical doctor belongs (a host side assistant) or a staff (a host side assistant).

### (Regarding to Search Processing)

In Embodiments 1 and 2 described above, it has been described that the processings of SA10 to SA19 (or SA15 to SA19), SB11 to SB17 (or SB14 to SB17), and SC11 to SC15, are performed, but the processings are not limited thereto. For example, the processings of SA10 to SA19 (or SA15 to SA19), SB11 to SB17 (or SB14 to SB17), and SC11 to SC15, may be omitted.

In addition, in Embodiments 1 and 2 described above, it has been described that in SB2, the diagnosis information included in the information such as diagnosis, received in SB1, is provided to the plurality of host side devices 10 corresponding to the host identification information stored in the host summary DB 33a, but the diagnosis information is not limited thereto. For example, the diagnosis information may be provided to the plurality of host side devices 10 corresponding to search condition information transmitted from the client side device 20, from the plurality of host side devices 10 corresponding to the host identification information (or a plurality of other host side devices 10, or the host side device 10 in which the plurality of other host side devices 10 are combined). Here, the "search condition information" is information indicating a condition for searching the host providing the service, and for example, is a concept including accident and sickness information indicating accident and sickness of the patient, symptomatic state information indicating a symptomatic state of the patient, attribution information indicating the attribution of the patient, or position information indicating the position of the patient relevant to the position of the medical doctor. In addition, in such a case, a provision method of the diagnosis information is arbitrary, and for example, the search may be performed as follows. That is, the information provision device 30 may include a search information storage section storing the search condition information and the host identification information in association with each other, and in a case where the diagnosis information and the search condition information are received by the communication unit 31, the host identification information corresponding to the search condition information may be specified, from the host identification information stored in the search information storage section, and the diagnosis information may be provided to the host side device 10 corresponding to the specified host identification information. Accordingly, it is possible to present the diagnosis information to the medical doctor according to the needs of the user, and to accurately search the host who meets the needs of the client.

In addition, in Embodiments 1 and 2 described above, it has been described that in SC4, the compensation information and the provision propriety information are generated, but it is not limited thereto, and for example, the generation of at least one of the compensation information and the provision propriety information may be omitted.

In addition, in Embodiments 1 and 2 described above, it has been described that in SB11, in a case where the information such as a first request, is received, in SB13, the host specification information is provided to the client side device 20, but it is not limited thereto. For example, in SB13, client specification information may be provided to the host side device 10 corresponding to the host specification information, in addition to the provision of the host specification information to the client side device 20. Here, the "client specification information" is information for specifying the patient, and in Embodiments 1 and 2, includes client name information indicating the name of the patient, client address information indicating the address of the patient, client email address information indicating an email address of the patient, and the like (here, the client specification information is not limited thereto, and for example, may include any one (or any two) of the client name information, the client address information, and the client email address information). Accordingly, it is possible to search the host who meets the needs of the client, and to further provide the optimal service to the client.

In addition, in Embodiments 1 and 2 described above, it has been described that in SB17, the response information received from the host side device 10 is provided to the client side device 20 or the device relevant to the host side device 10, but it is not limited thereto. For example, in SB15 described above, in a case where the information such as a second request, is provided to the device relevant to the host side device 10, and thus, the response information is received from the device relevant to the host side device 10, the response information may be provided to the client side device 20 and the host side device 10.

In addition, in Embodiments 1 and 2 described above, it has been described that the search processing of the information provision device 30 is ended after the processing of SB17, but it is not limited thereto. For example, progress information may be provided to the host side device 10 corresponding to the proposal intention information received in SB3 after the processing of SB17, and thus, the search processing may be ended. Here, the "progress information" is information indicating the progress of the search processing, and for example, is a concept including information of the number of people representing an intention, information of the number of specified people, and information of the number of matched people. Among them, the "information of the number of people representing an intention" is information indicating the number of medical doctors representing an intention of performing the proposal of the service, and for example, information indicating the total number of proposal intention information items provided in SB5, is generated as the information of the number of people representing an intention. In addition, the "information of the number of specified people" is information indicating the number of medical doctors specified by the patient, from the medical doctors representing the intention of performing the proposal of the service, and for example, information indicating the total number of host identification information items received in SB6, is generated as the information of the number of specified people. In addition, the "information of the number of matched people" is information indicating the number of medical doctors matched to the needs of the patient, from the medical doctors performing the proposal of the service, and for example, information indicating the total number of host side devices 10 to which the second request information is provided in SB15, is generated as the information of the number of matched people. Accordingly, it is possible to present the progress information to the host side device 10, and for example, it is possible for the medical doctor to confirm later that the patient has received a service provided by a medical doctor other than the medical doctor himself/herself.

### (Regarding to Past Aggregated Information)

In Embodiment 2 described above, it has been described that in a case where the questionnaire response information is received by the communication unit 31 at the time of generating the past aggregated information, the responses of the questionnaire response information items stored in the past questionnaire response DB 33c are aggregated, according to items such as the type of service corresponding to the received questionnaire response information, the age of the client, the gender of the client, or the host, and the information indicating the number of responses aggregated for each type of response, or the rate thereof, is generated as the past aggregated information to be generated, but it is not limited thereto. For example, in a case where the service type information indicating the type of medical procedure of the medical service, the client identification information, the client location information, the host medical facility information, and the host location information, are further stored in the past questionnaire response DB 33c, the responses of the questionnaire response information items are aggregated according to items such as the type of medical procedure of the medical service, the client, the resident area of the client, the medical facility to which the host belongs, or the location in which the medical facility exists (as an example, one item, or a combination of a plurality of items), and the information indicating the number of responses aggregated for each type of response, or the rate thereof, may be generated as the past aggregated information to be generated.

### (Notes)

A search system described in Note 1 is a search system searching a host providing a service to a client, the system comprises: a plurality of host side devices; a client side device; and an information provision system connected to each of the plurality of host side devices and the client side device to be capable of communicating with each other, wherein the client side device includes, a client side communication section for communicating with the information provision system or the plurality of host side devices, a client side output section outputting various information items relevant to the client side device, and an acquisition section acquiring diagnosis information indicating diagnosis contents of a diagnosis target, each of the plurality of host side devices includes, a host side communication section for communicating with the information provision system or the client side device, a host side output section outputting various information items relevant to each of the host side devices, a proposal intention information generation section generating proposal intention information indicating that there is an intention of performing a proposal of the service according to the diagnosis contents of the diagnosis target, and a proposal information generation section generating proposal information indicating proposal contents of the service, the information provision system includes, a communication section for communicating with the plurality of host side devices or the client side device, a host summary information storage section storing host identification information for uniquely identifying the host and host summary information indicating a summary of the host in association with each other, a provision section providing various information items to the plurality of host side devices or the client side device, and a notification information generation section generating notification information for notifying that it is a host performing the proposal of the service, the client side communication section of the client side device transmits the diagnosis information acquired by the acquisition section, to the information provision system, in a case in which the diagnosis information is received by the communication section, the provision section of the information provision system provides the diagnosis information to the plurality of host side devices, in a case in which a predetermined timing arrives after the diagnosis information provided by the provision section is output by the host side output section, the host side communication section of each of the plurality of host side devices transmits information including the proposal intention information generated by the proposal intention information generation section and the host identification information corresponding to the host side device to the information provision system, in a case in which the information including the proposal intention information and the host identification information is received by the communication section, the provision section of the information provision system extracts the host summary information corresponding to the host identification information from the host summary information stored in the host summary information storage section and provides information including the extracted host summary information and the corresponding host identification information to the client side device, the host summary information is output by the client side output section from the information including the host summary information and the host identification information provided by the provision section, and then, the client side communication section of the client side device transmits the host identification information corresponding to the host summary information specified by a predetermined method from the output host summary information to the information provision system, in a case in which the corresponding host identification information is received by the communication section, the provision section of the information provision system provides the notification information generated by the notification information generation section to the host side device corresponding to the host identification information, in a case in which a predetermined timing arrives after the notification information provided by the provision section is output by the host side output section, the host side communication section of each of the plurality of host side devices transmits the proposal information generated by the proposal information generation section to the information provision system, in a case in which the proposal information is received by the communication section, the provision section of the information provision system provides the proposal information to the client side device, and the proposal information is provided by the provision section, and then, the client side output section of the client side device outputs the proposal information.

Moreover, an information provision system described in Note 2 is an information provision system connected to each of a plurality of host side devices and a client side device to be capable of communicating with each other, the system comprises: a communication section for communicating with the plurality of host side devices or the client side device; a host summary information storage section storing host identification information for uniquely identifying a host providing a service to a client and host summary information indicating a summary of the host in association with each other; a provision section providing various information items to the plurality of host side devices or the client side device; and a notification information generation section generating notification information for notifying that it is a host performing a proposal of the service with respect to the client, wherein in a case in which diagnosis information indicating diagnosis contents of a diagnosis target is received by the communication section, the provision section provides the diagnosis information to the plurality of host side devices, in a case in which information including proposal intention information indicating that there is an intention of performing the proposal of the service according to the diagnosis contents of the diagnosis target and the host identification information is received by the communication section, the provision section extracts the host summary information corresponding to the host identification information from the host summary information stored in the host summary information storage section and provides information including the extracted host summary information and the corresponding host identification information to the client side device, in a case in which the host identification information corresponding to the host summary information specified by the client side device is received by the communication section, the provision section of the information provision system provides the notification information generated by the notification information generation section to the host side device corresponding to the host identification information, and in a case in which proposal information indicating proposal contents of the service is received by the communication section, the provision section of the information provision system provides the proposal information to the client side device.

Moreover, the information provision system described in Note 3 is the information provision system according to Note 2, wherein in a case in which information including the proposal intention information, compensation information indicating a compensation required at least for the proposal of the service or provision propriety information indicating whether or not to provide the proposed service to the client, and the host identification information is received by the communication section, the provision section extracts the host summary information corresponding to the received host identification information from the host summary information stored in the host summary information storage section and provides information including the extracted host summary information, the corresponding host identification information, and the received compensation information or the provision propriety information to the client side device.

Moreover, the information provision system described in Note 4 is the information provision system according to Note 2 or 3, wherein in a case in which questionnaire response information indicating a response of the host with respect to a service questionnaire is received by the communication section, the provision section provides the received questionnaire response information to the client side device.

Moreover, the information provision system described in Note 5 is the information provision system according to Note 4, further comprises: an aggregated information generation section generating aggregated information in which responses of a plurality of questionnaire response information items are aggregated in a case in which the plurality of questionnaire response information items are received by the communication section, wherein the plurality of questionnaire response information items are received, and then, the provision section provides the aggregated information generated by the aggregated information generation section to the client side device or the plurality of host side devices.

Moreover, the information provision system described in Note 6 is the information provision system according to Note 5, further comprises: a past questionnaire response information storage section storing the questionnaire response information received in the past, wherein in a case in which the questionnaire response information is received by the communication section, the aggregated information generation section generates past aggregated information in which the responses of the questionnaire response information items stored in the past questionnaire response information storage section are aggregated according to a predetermined item corresponding to the received questionnaire response information, and the questionnaire response information is received, and then, the provision section provides the past aggregated information generated by the aggregated information generation section to the client side device or the plurality of host side devices.

Moreover, the information provision system described in Note 7 is the information provision system according to any one of Notes 2 to 6, further comprises: a host specification information storage section storing the host identification information and host specification information for specifying the host in association with each other, wherein the proposal information is provided, and then, in a case in which information including first request information of requesting provision of the host specification information and the host identification information corresponding to the proposal information is received by the communication section, the provision section provides host specification information corresponding to the host identification information from the host specification information stored in the host specification information storage section to the client side device.

Moreover, the information provision system described in Note 8 is the information provision system according to Note 7, wherein the host specification information is provided, and then, in a case in which information including second request information of requesting a reservation of provision of the service and the host identification information corresponding to the host specification information is received by the communication section, the provision section provides the second request information at least to the host side device corresponding to the host identification information, and the second request information is provided, and then, in a case in which response information indicating whether or not a reservation with respect to the second request information is received is received by the communication section, the provision section provides the response information at least to the client side device.

Moreover, the information provision system described in Note 9 is the information provision system according to any one of Notes 2 to 8, wherein the service is a medical service, the diagnosis target is the client, and the diagnosis information includes information indicating a medical certificate of the client, information indicating a medical information provision document of the client, or information indicating a list of differential diagnosis of the client.

Moreover, the information provision system described in Note 10 is the information provision system according to any one of Notes 2 to 9, wherein the host summary information includes actual performance information indicating actual performance of the host, evaluation information which becomes an index at the time of evaluating the host, or background information indicating a background of the host.

Moreover, a client side device described in Note 11 is a client side device connected to each of an information provision system and a plurality of host side devices to be capable of communicating with each other, the device comprises: a client side communication section for communicating with the information provision system or the plurality of host side devices; a client side output section outputting various information items relevant to the client side device; and an acquisition section acquiring diagnosis information indicating diagnosis contents of a diagnosis target, wherein the client side communication section transmits the diagnosis information acquired by the acquisition section, to the information provision system, host summary information is output by the client side output section from information which is provided from the information provision system and includes the host summary information indicating a summary of a host providing a service to a client and host identification information for uniquely identifying the host, and then, the client side communication section transmits the host identification information corresponding to the host summary information specified by a predetermined method, from the output host summary information, to the information provision system, and proposal information indicating proposal contents of the service is provided from the information provision system, and then, the client side output section outputs the proposal information.

Moreover, a host side device described in Note 12 is a host side device connected to a client side device and an information provision system to be capable of communicating with each other, the device comprises: a host side communication section for communicating with the information provision system or the client side device; a host side output section outputting various information items relevant to the host side device; a proposal intention information generation section generating proposal intention information indicating that there is an intention of performing a proposal of a service with respect to a client according to diagnosis contents of a diagnosis target; and a proposal information generation section generating proposal information indicating proposal contents of the service, wherein in a case in which a predetermined timing arrives after diagnosis information provided from the information provision system and indicating the diagnosis contents of the diagnosis target is output by the host side output section, the host side communication section transmits information including the proposal intention information generated by the proposal intention information generation section and host identification information corresponding to the host side device for uniquely identifying a host providing the service to a client to the information provision system, and in a case in which a predetermined timing arrives after notification information provided from the information provision system for notifying that it is a host performing proposal of the service with respect to the client is output by the host side output section, the host side communication section transmits the proposal information generated by the proposal information generation section to the information provision system.

Moreover, an information provision method described in Note 13 is an information provision method to be performed in an information provision system connected to each of a plurality of host side devices and a client side device to be capable of communicating with each other, the method comprises: a communication step of allowing a communication section to communicate with the plurality of host side devices or the client side device; a provision step of allowing a provision section to provide various information items to the plurality of host side devices or the client side device; and a notification information generation step of allowing a notification information generation section to generate notification information for notifying that it is a host performing a proposal of a service to a client, wherein in the provision step, in a case in which diagnosis information indicating diagnosis contents of a diagnosis target is received in the communication step, the diagnosis information is provided to the plurality of host side devices, in a case in which information including proposal intention information indicating that there is an intention of performing the proposal of the service according to the diagnosis contents of the diagnosis target and host identification information for uniquely identifying the host is received in the communication step, host summary information corresponding to the host identification information is extracted from the host summary information indicating a summary of the host, which is stored in a host summary information storage section, in association with the host identification information, and information including the extracted host summary information and the corresponding host identification information is provided to the client side device, in a case in which the host summary information specified by the client side device is received in the communication step, the notification information generated by the notification information generation section is provided to the host side device corresponding to the host summary information, and in a case in which proposal information indicating proposal contents of the service is received in the communication step, the proposal information is provided to the client side device.

Moreover, an information provision program method described in Note 14 is an information provision program to be executed in an information provision system connected to each of a plurality of host side devices and a client side device to be capable of communicating with each other, the program allowing a computer to function as: a communication section for communicating with the plurality of host side devices or the client side device; a host summary information storage section storing host identification information for uniquely identifying a host providing a service to a client and host summary information indicating a summary of the host in association with each other; a provision section providing various information items to the plurality of host side devices or the client side device; and a notification information generation section generating notification information for notifying that it is a host performing a proposal of a service with respect to a client, wherein in a case in which diagnosis information indicating diagnosis contents of a diagnosis target is received by the communication section, the provision section provides the diagnosis information to the plurality of host side devices, in a case in which information including proposal intention information indicating that there is an intention of performing the proposal of the service according to the diagnosis contents of the diagnosis target, and the host identification information is received by the communication section, the provision section extracts the host summary information corresponding to the host identification information from the host summary information stored in the host summary information storage section and provides information including the extracted host summary information and the corresponding host identification information to the client side device, in a case in which the host identification information corresponding to the host summary information specified by the client side device is received by the communication section, the provision section of the information provision system provides the notification information generated by the notification information generation section to the host side device corresponding to the host identification information, and in a case in which proposal information indicating proposal contents of the service is received by the communication section, the provision section of the information provision system provides the proposal information to the client side device.

Moreover, a client side program method described in Note 15 is a client side program to be executed in a client side device connected to each of an information provision system and a plurality of host side devices to be capable of communicating with each other, the program allowing a computer to function as: a client side communication section for communicating with the information provision system or the plurality of host side devices; a client side output section outputting various information items relevant to the client side device; and an acquisition section acquiring diagnosis information indicating diagnosis contents of a diagnosis target, wherein the client side communication section transmits the diagnosis information acquired by the acquisition section, to the information provision system, host summary information is output by the client side output section from information which is provided from the information provision system and includes the host summary information indicating a summary of a host providing a service to a client and host identification information for uniquely identifying the host, and then, the client side communication section transmits the host identification information corresponding to the host summary information specified by a predetermined method, from the output host summary information, to the information provision system, and proposal information indicating proposal contents of the service is provided from the information provision system, and then, the client side output section outputs the proposal information.

Moreover, a host side program method described in Note 16 is a host side program to be executed in a host side device connected to a client side device and an information provision system to be capable of communicating with each other, the program allowing a computer to function as: a host side communication section for communicating with the information provision system or the client side device; a host side output section outputting various information items relevant to the host side device; a proposal intention information generation section generating proposal intention information indicating that there is an intention of performing a proposal of a service with respect to a client according to diagnosis contents of a diagnosis target; and a proposal information generation section generating proposal information indicating proposal contents of the service, wherein in a case in which a predetermined timing arrives after diagnosis information provided from the information provision system and indicating the diagnosis contents of the diagnosis target is output by the host side output section, the host side communication section transmits information including the proposal intention information generated by the proposal intention information generation section, and host identification information which corresponds to the host side device for uniquely identifying a host providing the service to a client to the information provision system, and in a case in which a predetermined timing arrives after notification information which is provided from the information provision system for notifying that it is a host performing proposal of the service with respect to the client is output by the host side output section, the host side communication section transmits the proposal information generated by the proposal information generation section, to the information provision system.

### (Advantageous Effect of Notes)

According to the search system described in Note 1, the information provision system in Note 2, the client side device in Note 11, the host side device described in Note 12, the information provision method described in Note 13, the information provision program described in Note 14, the client side program described in Note 15, and the host side program described in Note 16, in a case where the provision section of the information provision system provides the diagnosis information to the plurality of host side devices, and in a case where a predetermined timing arrives after the diagnosis information is output by the host side output section, the host side communication section of each of the plurality of host side devices transmits information including the proposal intention information, and the host identification information corresponding to the host side device, to the information provision device, and in a case where the information including the proposal intention information and the host identification information, is received, the provision section of the information provision device extracts the host summary information corresponding to the host identification information, from the host summary information storage section, and provides information including the extracted host summary information and the corresponding host identification information, to the client side device, and the host summary information is output by the client side output section, and then, the client side communication section of the client side device transmits the host identification information corresponding to the specified host summary information, to the information provision device from the output host summary information, by a predetermined method, and in a case where the corresponding host identification information is received, the provision section of the information provision device provides the notification information to the host side device corresponding to the host identification information, and in a case where a predetermined timing arrives after the notification information is output by the host side output section, the host side communication section of each of the plurality of host side devices transmits the proposal information to the information provision device, and in a case where the proposal information is received, the provision section of the information provision device provides the proposal information to the client side device, and the client side output section of the client side device outputs the proposal information after the proposal information is provided, and thus, it is possible to present various proposal information items to the client. Accordingly, it is possible for the client to receive the service corresponding to the proposal information which meets the needs, from various proposal information items, and it is possible to provide the optimal service to the client.

According to the information provision system described in Note 3, in a case where information including the compensation information or the provision propriety information, and the host identification information, is received, the provision section extracts the host summary information corresponding to the received host identification information, from the host summary information stored in the host summary information storage section, and provides information including the extracted host summary information, the corresponding host identification information, and the received compensation information or provision propriety information, to the client side device, and thus, it is possible to present the compensation information and the provision propriety information, included in the information such as a host summary, to the client, and to easily search the host who meets the needs of the client.

According to the information provision system described in Note 4, in a case where the questionnaire response information is received by the communication section, the provision section provides the received questionnaire response information to the client side device, and thus, it is possible to present the questionnaire response information reflecting the opinion with respect to the service of the host, to the client. Accordingly, it is possible for the client to easily search the host who meets the needs of the client, on the basis of the questionnaire response information, and thus, it is possible to prevent the provision of the service which does not meet the needs of a user, from being received, or the host from being searched again.

According to the information provision system described in Note 5, the plurality of questionnaire response information items are received, and then, the provision section provides the aggregated information generated by the aggregated information generation section, to the client side device or the plurality of host side devices, and thus, it is possible to present the aggregated information reflecting the opinion of the plurality of hosts, to the client. Accordingly, the aggregated information can be treated as the determination standard at the time of searching the host, and thus, it is possible for the client to easily search the host who meets the needs of the client, on the basis of the aggregated information. In addition, it is possible to present the aggregated information to the plurality of hosts, and it is possible for the plurality of hosts to objectively grasp a standing position with respect to the responses of the questionnaires of themselves.

According to the information provision system described in Note 6, the questionnaire response information is received, and then, the provision section provides the past aggregated information generated by the aggregated information generation section, to the client side device or the plurality of host side devices, and thus, it is possible to present the past aggregated information to the client, and to treat the past aggregated information as the determination standard at the time of searching the host, and therefore, it is possible for the client to easily search the host who meets the needs of the client, on the basis of the past aggregated information. In addition, it is possible to present the past aggregated information to the plurality of hosts, and it is possible for the plurality of hosts to more objectively grasp the standing position with respect to the responses of the questionnaires of themselves.

According to the information provision system described in Note 7, in a case where the proposal information is provided, and then, information including the first request information, and the host identification information corresponding to the proposal information, is received, the provision section provides the host specification information corresponding to the host identification information, to the client side device, from the host specification information stored in the host specification information storage section, and thus, it is possible to present the host specification information to the client, and for example, it is possible for the client to reliably contact with the host providing the service.

According to the information provision system described in Note 8, in a case where the host specification information is provided, and then, information including the second request information, and the host identification information corresponding to the host specification information, is received, the provision section provides the second request information at least to the host side device corresponding to the host identification information, and in a case where the second request information is provided, and then, the response information is received, the provision section provides the response information at least to the client side device, and thus, it is possible to present the response information at least to the client. Accordingly, it is possible to reserve the provision of the service without allowing the client to visit the facility providing the service, and to reliably receive the service from the host who provides the service.

According to the information provision system described in Note 9, the service is the medical service, the diagnosis target is the client, and the diagnosis information includes the information indicating the medical certificate of the client, the information indicating the medical information provision document of the client, or the information indicating the list of the differential diagnosis of the client, and thus, it is possible to present such information items to the host, and it is possible for the host to grasp the diagnosis contents of the client in detail.

According to the information provision system described in Note 10, the host summary information includes the actual performance information, the evaluation information, or the background information, and thus, it is possible to present such information items to the client, and to grasp the summary of the host in detail.

### Reference Signs List

- 1: Search system
- 2: Network
- 3: Medical facility
- 10: Host side device
- 11: Host side communication unit
- 12: Touch pad
- 13: Display
- 14: Speaker
- 15: Control unit
- 15a: Proposal intention information generation unit
- 15b: Proposal information generation unit
- 15c: Response information generation unit
- 16: Data recording unit
- 20: Client side device
- 21: Client side communication unit
- 22: Touch pad
- 23: Display
- 24: Speaker
- 25: Control unit
- 25a: Acquisition unit
- 25b: Request information generation unit
- 26: Data recording unit
- 30: Information provision device
- 31: Communication unit
- 32: Control unit
- 32a: Provision unit
- 32b: Notification information generation unit
- 32c: Aggregated information generation unit
- 33: Data recording unit
- 33a: Host summary DB
- 33b: Host specification DB
- 33c: Past questionnaire response DB

## Claims

1. A search system searching a host providing a service to a client, the system comprising:
a plurality of host side devices;
a client side device; and
an information provision system connected to each of the plurality of host side devices and the client side device to be capable of communicating with each other,
wherein the client side device includes,
a client side communication section for communicating with the information provision system or the plurality of host side devices,
a client side output section outputting various information items relevant to the client side device, and
an acquisition section acquiring diagnosis information indicating diagnosis contents of a diagnosis target,
each of the plurality of host side devices includes,
a host side communication section for communicating with the information provision system or the client side device,
a host side output section outputting various information items relevant to each of the host side devices,
a proposal intention information generation section generating proposal intention information indicating that there is an intention of performing a proposal of the service according to the diagnosis contents of the diagnosis target, and
a proposal information generation section generating proposal information indicating proposal contents of the service,
the information provision system includes,
a communication section for communicating with the plurality of host side devices or the client side device,
a host summary information storage section storing host identification information for uniquely identifying the host and host summary information indicating a summary of the host in association with each other,
a provision section providing various information items to the plurality of host side devices or the client side device, and
a notification information generation section generating notification information for notifying that it is a host performing the proposal of the service,
the client side communication section of the client side device transmits the diagnosis information acquired by the acquisition section, to the information provision system,
in a case in which the diagnosis information is received by the communication section, the provision section of the information provision system provides the diagnosis information to the plurality of host side devices,
in a case in which a predetermined timing arrives after the diagnosis information provided by the provision section is output by the host side output section, the host side communication section of each of the plurality of host side devices transmits information including the proposal intention information generated by the proposal intention information generation section and the host identification information corresponding to the host side device to the information provision system,
in a case in which the information including the proposal intention information and the host identification information is received by the communication section, the provision section of the information provision system extracts the host summary information corresponding to the host identification information from the host summary information stored in the host summary information storage section and provides information including the extracted host summary information and the corresponding host identification information to the client side device,
the host summary information is output by the client side output section from the information including the host summary information and the host identification information provided by the provision section, and then, the client side communication section of the client side device transmits the host identification information corresponding to the host summary information specified by a predetermined method from the output host summary information to the information provision system,
in a case in which the corresponding host identification information is received by the communication section, the provision section of the information provision system provides the notification information generated by the notification information generation section to the host side device corresponding to the host identification information,
in a case in which a predetermined timing arrives after the notification information provided by the provision section is output by the host side output section, the host side communication section of each of the plurality of host side devices transmits the proposal information generated by the proposal information generation section to the information provision system,
in a case in which the proposal information is received by the communication section, the provision section of the information provision system provides the proposal information to the client side device, and
the proposal information is provided by the provision section, and then, the client side output section of the client side device outputs the proposal information.

2. An information provision system connected to each of a plurality of host side devices and a client side device to be capable of communicating with each other, the system comprising:
a communication section for communicating with the plurality of host side devices or the client side device;
a host summary information storage section storing host identification information for uniquely identifying a host providing a service to a client and host summary information indicating a summary of the host in association with each other;
a provision section providing various information items to the plurality of host side devices or the client side device; and
a notification information generation section generating notification information for notifying that it is a host performing a proposal of the service with respect to the client,
wherein in a case in which diagnosis information indicating diagnosis contents of a diagnosis target is received by the communication section, the provision section provides the diagnosis information to the plurality of host side devices,
in a case in which information including proposal intention information indicating that there is an intention of performing the proposal of the service according to the diagnosis contents of the diagnosis target and the host identification information is received by the communication section, the provision section extracts the host summary information corresponding to the host identification information from the host summary information stored in the host summary information storage section and provides information including the extracted host summary information and the corresponding host identification information to the client side device,
in a case in which the host identification information corresponding to the host summary information specified by the client side device is received by the communication section, the provision section of the information provision system provides the notification information generated by the notification information generation section to the host side device corresponding to the host identification information, and
in a case in which proposal information indicating proposal contents of the service is received by the communication section, the provision section of the information provision system provides the proposal information to the client side device.

3. The information provision system according to claim 2,
wherein in a case in which information including the proposal intention information, compensation information indicating a compensation required at least for the proposal of the service or provision propriety information indicating whether or not to provide the proposed service to the client, and the host identification information is received by the communication section, the provision section extracts the host summary information corresponding to the received host identification information from the host summary information stored in the host summary information storage section and provides information including the extracted host summary information, the corresponding host identification information, and the received compensation information or the provision propriety information to the client side device.

4. The information provision system according to claim 2 or 3,
wherein in a case in which questionnaire response information indicating a response of the host with respect to a service questionnaire is received by the communication section, the provision section provides the received questionnaire response information to the client side device.

5. The information provision system according to claim 4, further comprising:
an aggregated information generation section generating aggregated information in which responses of a plurality of questionnaire response information items are aggregated in a case in which the plurality of questionnaire response information items are received by the communication section,
wherein the plurality of questionnaire response information items are received, and then, the provision section provides the aggregated information generated by the aggregated information generation section to the client side device or the plurality of host side devices.

6. The information provision system according to claim 5, further comprising:
a past questionnaire response information storage section storing the questionnaire response information received in the past,
wherein in a case in which the questionnaire response information is received by the communication section, the aggregated information generation section generates past aggregated information in which the responses of the questionnaire response information items stored in the past questionnaire response information storage section are aggregated according to a predetermined item corresponding to the received questionnaire response information, and
the questionnaire response information is received, and then, the provision section provides the past aggregated information generated by the aggregated information generation section to the client side device or the plurality of host side devices.

7. The information provision system according to any one of claims 2 to 6, further comprising:
a host specification information storage section storing the host identification information and host specification information for specifying the host in association with each other,
wherein the proposal information is provided, and then, in a case in which information including first request information of requesting provision of the host specification information and the host identification information corresponding to the proposal information is received by the communication section, the provision section provides host specification information corresponding to the host identification information from the host specification information stored in the host specification information storage section to the client side device.

8. The information provision system according to claim 7,
wherein the host specification information is provided, and then, in a case in which information including second request information of requesting a reservation of provision of the service and the host identification information corresponding to the host specification information is received by the communication section, the provision section provides the second request information at least to the host side device corresponding to the host identification information, and
the second request information is provided, and then, in a case in which response information indicating whether or not a reservation with respect to the second request information is received is received by the communication section, the provision section provides the response information at least to the client side device.

9. The information provision system according to any one of claims 2 to 8,
wherein the service is a medical service,
the diagnosis target is the client, and
the diagnosis information includes information indicating a medical certificate of the client, information indicating a medical information provision document of the client, or information indicating a list of differential diagnosis of the client.

10. The information provision system according to any one of claims 2 to 9,
wherein the host summary information includes actual performance information indicating actual performance of the host, evaluation information which becomes an index at the time of evaluating the host, or background information indicating a background of the host.

11. A client side device connected to each of an information provision system and a plurality of host side devices to be capable of communicating with each other, the device comprising:
a client side communication section for communicating with the information provision system or the plurality of host side devices;
a client side output section outputting various information items relevant to the client side device; and
an acquisition section acquiring diagnosis information indicating diagnosis contents of a diagnosis target,
wherein the client side communication section transmits the diagnosis information acquired by the acquisition section, to the information provision system,
host summary information is output by the client side output section from information which is provided from the information provision system and includes the host summary information indicating a summary of a host providing a service to a client and host identification information for uniquely identifying the host, and then, the client side communication section transmits the host identification information corresponding to the host summary information specified by a predetermined method, from the output host summary information, to the information provision system, and
proposal information indicating proposal contents of the service is provided from the information provision system, and then, the client side output section outputs the proposal information.

12. A host side device connected to a client side device and an information provision system to be capable of communicating with each other, the device comprising:
a host side communication section for communicating with the information provision system or the client side device;
a host side output section outputting various information items relevant to the host side device;
a proposal intention information generation section generating proposal intention information indicating that there is an intention of performing a proposal of a service with respect to a client according to diagnosis contents of a diagnosis target; and
a proposal information generation section generating proposal information indicating proposal contents of the service,
wherein in a case in which a predetermined timing arrives after diagnosis information provided from the information provision system and indicating the diagnosis contents of the diagnosis target is output by the host side output section, the host side communication section transmits information including the proposal intention information generated by the proposal intention information generation section and host identification information corresponding to the host side device for uniquely identifying a host providing the service to a client to the information provision system, and
in a case in which a predetermined timing arrives after notification information provided from the information provision system for notifying that it is a host performing proposal of the service with respect to the client is output by the host side output section, the host side communication section transmits the proposal information generated by the proposal information generation section to the information provision system.

13. An information provision method to be performed in an information provision system connected to each of a plurality of host side devices and a client side device to be capable of communicating with each other, the method comprising:
a communication step of allowing a communication section to communicate with the plurality of host side devices or the client side device;
a provision step of allowing a provision section to provide various information items to the plurality of host side devices or the client side device; and
a notification information generation step of allowing a notification information generation section to generate notification information for notifying that it is a host performing a proposal of a service to a client,
wherein in the provision step,
in a case in which diagnosis information indicating diagnosis contents of a diagnosis target is received in the communication step, the diagnosis information is provided to the plurality of host side devices,
in a case in which information including proposal intention information indicating that there is an intention of performing the proposal of the service according to the diagnosis contents of the diagnosis target and host identification information for uniquely identifying the host is received in the communication step, host summary information corresponding to the host identification information is extracted from the host summary information indicating a summary of the host, which is stored in a host summary information storage section, in association with the host identification information, and information including the extracted host summary information and the corresponding host identification information is provided to the client side device,
in a case in which the host summary information specified by the client side device is received in the communication step, the notification information generated by the notification information generation section is provided to the host side device corresponding to the host summary information, and
in a case in which proposal information indicating proposal contents of the service is received in the communication step, the proposal information is provided to the client side device.

14. An information provision program to be executed in an information provision system connected to each of a plurality of host side devices and a client side device to be capable of communicating with each other, the program allowing a computer to function as:
a communication section for communicating with the plurality of host side devices or the client side device;
a host summary information storage section storing host identification information for uniquely identifying a host providing a service to a client and host summary information indicating a summary of the host in association with each other;
a provision section providing various information items to the plurality of host side devices or the client side device; and
a notification information generation section generating notification information for notifying that it is a host performing a proposal of a service with respect to a client,
wherein in a case in which diagnosis information indicating diagnosis contents of a diagnosis target is received by the communication section, the provision section provides the diagnosis information to the plurality of host side devices,
in a case in which information including proposal intention information indicating that there is an intention of performing the proposal of the service according to the diagnosis contents of the diagnosis target, and the host identification information is received by the communication section, the provision section extracts the host summary information corresponding to the host identification information from the host summary information stored in the host summary information storage section and provides information including the extracted host summary information and the corresponding host identification information to the client side device,
in a case in which the host identification information corresponding to the host summary information specified by the client side device is received by the communication section, the provision section of the information provision system provides the notification information generated by the notification information generation section to the host side device corresponding to the host identification information, and
in a case in which proposal information indicating proposal contents of the service is received by the communication section, the provision section of the information provision system provides the proposal information to the client side device.

15. A client side program to be executed in a client side device connected to each of an information provision system and a plurality of host side devices to be capable of communicating with each other, the program allowing a computer to function as:
a client side communication section for communicating with the information provision system or the plurality of host side devices;
a client side output section outputting various information items relevant to the client side device; and
an acquisition section acquiring diagnosis information indicating diagnosis contents of a diagnosis target,
wherein the client side communication section transmits the diagnosis information acquired by the acquisition section, to the information provision system,
host summary information is output by the client side output section from information which is provided from the information provision system and includes the host summary information indicating a summary of a host providing a service to a client and host identification information for uniquely identifying the host, and then, the client side communication section transmits the host identification information corresponding to the host summary information specified by a predetermined method, from the output host summary information, to the information provision system, and
proposal information indicating proposal contents of the service is provided from the information provision system, and then, the client side output section outputs the proposal information.

16. A host side program to be executed in a host side device connected to a client side device and an information provision system to be capable of communicating with each other, the program allowing a computer to function as:
a host side communication section for communicating with the information provision system or the client side device;
a host side output section outputting various information items relevant to the host side device;
a proposal intention information generation section generating proposal intention information indicating that there is an intention of performing a proposal of a service with respect to a client according to diagnosis contents of a diagnosis target; and
a proposal information generation section generating proposal information indicating proposal contents of the service,
wherein in a case in which a predetermined timing arrives after diagnosis information provided from the information provision system and indicating the diagnosis contents of the diagnosis target is output by the host side output section, the host side communication section transmits information including the proposal intention information generated by the proposal intention information generation section, and host identification information which corresponds to the host side device for uniquely identifying a host providing the service to a client to the information provision system, and
in a case in which a predetermined timing arrives after notification information which is provided from the information provision system for notifying that it is a host performing proposal of the service with respect to the client is output by the host side output section, the host side communication section transmits the proposal information generated by the proposal information generation section, to the information provision system.
